# EUROPEAN PATENT SPECIFICATION

(11) **EP 4 422 622 B1**
(45) Date of publication and mention of the grant of the patent: **18.06.2025**
(21) Application number: 23765573.3
(22) Date of filing: 31.07.2023
(51) Int. Cl.: A61K 31/417, A61K 31/216, A61P 17/00, A61K 38/05, A61K 45/06

(54) **GAMMA-SECRETASE INHIBITORS FOR USE IN THE TREATMENT OF INHERITED EPIDERMOLYSIS BULLOSA**
GAMMA-SEKRETASE-HEMMER ZUR VERWENDUNG BEI DER BEHANDLUNG VON VERERBTER EPIDERMOLYSIS BULLOSA
INHIBITEURS DE GAMMA-SÉCRÉTASE DESTINÉS À ÊTRE UTILISÉS DANS LE TRAITEMENT DE L'ÉPIDERMOLYSE BULLEUSE HÉRÉDITAIRE

(30) Priority: 02.08.2022 IT 202200016407
(43) Date of publication of application: 04.09.2024
(73) Proprietor: Ospedale Pediatrico Bambino Gesù, 00165 Roma (IT); Fondazione Luigi Maria Monti, 00167 Rome (IT)
(72) Inventor: CONDORELLI, Angelo Giuseppe, 00165 Roma (RM) (IT); ZAMBRUNO, Giovanna, 00165 Roma (RM) (IT); EL HACHEM, May, 00165 Roma (RM) (IT); CASTIGLIA, Daniele, 00167 Roma (RM) (IT); ODORISIO, Teresa, 00167 Roma (RM) (IT)
(74) Representative: Gitto, Serena
(86) International application number: PCT/IT2023/050186
(87) International publication number: WO 2024/028912

(56) References cited:
- WO-A1-2008/091222
- WO-A1-2020/208572
- POURANI MOHAMMAD REZA ET AL: "Losartan treatment improves recessive dystrophic epidermolysis bullosa: A case series", vol. 35, no. 7, 29 April 2022 (2022-04-29), US, pages 1 - 6, XP093021711, ISSN: 1396-0296, Retrieved from the Internet <URL:https://onlinelibrary.wiley.com/doi/full-xml/10.1111/dth.15515> DOI: 10.1111/dth.15515
- BERNASCONI ROCCO ET AL: "Pro-inflammatory immunity supports fibrosis advancement in epidermolysis bullosa: intervention with Ang-(1-7)", EMBO MOLECULAR MEDICINE, vol. 13, no. 10, 30 August 2021 (2021-08-30), US, pages 1 - 20, XP093021700, ISSN: 1757-4676, Retrieved from the Internet <URL:https://onlinelibrary.wiley.com/doi/full-xml/10.15252/emmm.202114392> DOI: 10.15252/emmm.202114392
- KENNETH R GOLDSCHNEIDER ET AL: "Pain care for patients with epidermolysis bullosa: best care practice guidelines", BMC MEDICINE, BIOMED CENTRAL LTD., LONDON, GB, vol. 12, no. 1, 9 October 2014 (2014-10-09), pages 178, XP021200264, ISSN: 1741-7015, DOI: 10.1186/S12916-014-0178-2
- CONDORELLI ANGELO GIUSEPPE ET AL: "Notch-ing up knowledge on molecular mechanisms of skin fibrosis: focus on the multifaceted Notch signalling pathway", vol. 28, no. 1, 9 May 2021 (2021-05-09), pages 1 - 17, XP093021525, Retrieved from the Internet <URL:https://link.springer.com/article/10.1186/s12929-021-00732-8/fulltext.html> DOI: 10.1186/s12929-021-00732-8
- ANONYMOUS: "Epidermolysis bullosa - NHS", 23 June 2021 (2021-06-23), XP093094611, Retrieved from the Internet <URL:https://www.nhs.uk/conditions/epidermolysis-bullosa/> [retrieved on 20231024]

## Description

The present invention concerns gamma-secretase inhibitors for use in the treatment of inherited epidermolysis bullosa. In particular, the present invention concerns gamma-secretase inhibitors for use in the treatment of inherited epidermolysis bullosa and/or fibrosis associated to inherited epidermolysis bullosa, in particular for use in the treatment of recessive dystrophic epidermolysis bullosa and recessive dystrophic epidermolysis bullosa-associated fibrosis.

It is known that the term epidermolysis bullosa usually refers to inherited epidermolysis bullosa (EB), which is a genetically determined disease [1]. Another type of epidermolysis bullosa is Epidermolysis Bullosa Acquisita (EBA), that is an acquired and autoimmune disease, mediated by circulating and tissue-bound autoantibodies (antibodies produced by plasma cells derived from B cells) targeting collagen VII [2]. With respect to inherited epidermolysis bullosa, EBA is characterised by different pathogenetic mechanisms. Therefore, inherited epidermolysis bullosa and epidermolysis bullosa acquisita are two distinct and different diseases.

Inherited epidermolysis bullosa (EB) is a group of skin fragility disorders determined by congenital defects in protein adhesion components of cutaneous basement membrane zone [1,3].

The recessive dystrophic subtype of EB (RDEB) is caused by loss-of-function mutations in the *COL7A1* gene, encoding for the collagen VII (COL7), which ensures skin integrity in response to traumatism and shear stress. Distinctive features of RDEB patients are unremitting mucocutaneous blisters and erosions, chronic wounds, and self-fueled inflammation and fibrosis. The latter results in highly disabling clinical sequelae comprising mitten deformities, joint contractures and esophageal strictures, and predisposes to the early onset of aggressive cutaneous squamous cell carcinomas, which represent the first cause of death in RDEB patients [4].

There is currently no cure for RDEB, and disease treatment relies on symptomatic measures, including wound care, nutritional support and surgery, by a multidisciplinary team [1,5,6]. As for wound care, the European Medicines Agency (EMA) authorized the use of Filsuvez (Amryt Pharmaceuticals DAC) - a gel containing dry extract from two species of birch bark - for the topical treatment of partial thickness wounds in adults and children aged 6 months or older affected with inherited EB, including patients with RDEB [7].

In the last years, approaches of gene-, cell- and protein-based therapy for COL7 replacement for RDEB have shown promise in preclinical studies, and some of them have entered or even completed phase I/II clinical trials [8-11]. In particular, ex-vivo gene therapy strategies using integrating retroviral or lentiviral vectors have been explored for their potential to bring durable clinical benefit to RDEB patients, provided epidermal stem cells are targeted [12]. Indeed, in a patient suffering from a different EB form, junctional EB, grafting of approximately 80% of skin surface with autologous epithelial sheets genetically corrected with a retroviral vector expressing the LAMB3 cDNA under the control of the viral LTR promoter regenerated a fully functional epidermis, which remained genetically corrected at 5-year follow-up [13,14]. However, a similar approach of grafting *COL7A1* retrovirally transduced autologous epidermal sheets to RDEB wounds resulted in variably increased COL7 expression and wound healing, which declined over time [15]. A topical gene therapy-based gel, commercialized with the name of Vyjuvek^{™} (Krystal Biotech Inc.), has been recently approved by the U.S. Food and Drug Administration (FDA) for the treatment of wounds in patients 6 months of age and older with dystrophic EB (DEB). Specifically, Vvjuvek^{™} contains a laboratory engineered herpes-simplex virus type 1 (HSV-1) which delivers normal copies of the *COL7A1* gene to the wounds, restoring type VII collagen protein levels at the treated sites and improving wound healing [16]. Despite its safety and efficacy, Vvjuvek^{™} is authorized for treatment of skin wounds only, and limitations apply to the total wound area that can be treated and maximum weekly dose administered. In addition, it presents issues concerning its application at difficult-to-treat body sites (*e.g.* periorificial areas, skin folds) and high costs.

Cell therapy strategies for RDEB also showed limited efficacy and tolerability, as in the case of local injections of allogeneic fibroblasts, and/or implied severe complications, as seen for bone marrow transplantation in these fragile patients [8].

Systemic protein therapy with human recombinant COL7 has been long advocated, but only recently entered phase I/II clinical trials (NCT03752905 and NCT05143190). Thus, to date curative therapies have turned out more challenging than originally expected and the unmet medical need in RDEB remains very high.

As a result, growing efforts are currently being focused on the development of disease-modifying therapies able to lessen or delay specific disease manifestations and complications [8].

RDEB-associated fibrosis is the result of a complex interplay among a wide range of molecular determinants including cytokines and growth factors, first and foremost the transforming growth factor-β1 (TGF-β1) [17,18], extracellular matrix components [19-22] and epigenetic factors [23,24]. The phenoconversion of a heterogeneous group of precursor cells, which mainly consists of local resident fibroblasts, into a highly specialized cell type called myofibroblast, is a regular event and a key pathogenetic mechanisms underlying every fibrotic condition, regardless of the affected tissue/organs [25,26]. Myofibroblast are cells endowed with a potent ability to secrete extracellular matrix (ECM) components, synthetize stress fibers and contractile proteins, and, in turn, to transmit mechanical forces to the ECM [25,26]. By reason of their features, myofibroblasts play a pivotal role in the context of wound healing and tissue remodeling. However, in patient affected with RDEB and other cutaneous fibrotic diseases, myofibroblasts are hyper-activated and chronically reside in the dermis, triggering and sustaining fibrosis in a self-catalyzed manner [27,28].

It is known that the Notch signaling cascade is an evolutionarily conserved pathway involved in a variety of biological processes in health and disease conditions, including skin fibrosis [29,30]. In general, Notch activation requires a physical interaction (trans-activation) between one of the four Notch receptors exposed by a "signal sending" cell and one of the five Notch ligand decorating a neighboring "signal receiving" cell [29]. Notch receptor-ligand engagement triggers two successive proteolytic cleavages, indicated as S2 and S3, led by ADAM family metalloproteases (*e.g.* ADAM10 and ADAM17) and by the gamma (γ)-secretase complex, respectively. The remodeling of Notch receptor determines the cytoplasmic release of the biologically active Notch intracellular domain (N-ICD) which translocates into the nucleus and, in cooperation with the ubiquitous transcription factors such as CBF-1/suppressor of hairless/Lag1 (CSL) and the co-activator MAML1, regulates gene transcription [29].

Some evidences indicate that Notch signaling is activated in fibrotic disorders of different tissues and organs, including the skin [30,31]. However, its role in RDEB fibrosis is almost unexplored. A previous study of the inventors showed that the protein levels of the Notch ligand Jagged-1 (JAG1) and the intracellular form of NOTCH1 (N1-ICD) are up-regulated in RDEB fibroblasts (RDEB-FBs) as compared to primary skin fibroblasts from healthy subjects (NH-FBs), and positively correlate with the abundance of the pro-fibrotic microRNA (miR), miR-145-5p [24].

WO 2008/091222 relates to methods for treating a disorder comprising administering an effective amount of a DLL4 agonist simultaneously blocking both Notch signalling and internalisation of DLL4 to a subject in need of such treatment.

In the light of the above, it is therefore apparent the need to provide new treatments for inherited epidermolysis bullosa, in particular recessive dystrophic epidermolysis bullosa, and the associated fibrosis, able to overcome the disadvantages of the known treatments.

According to the present invention, it has now been surprisingly found that two gamma-secretase inhibitors (GSIs), *i.e.* DAPT and PF-03084014 (nirogacestat), are able to counteract fibrotic behavior of recessive dystrophic epidermolysis bullosa fibroblasts.

WO 2020/208572 relates to a combination therapy of a pharmaceutically active antigen binding protein, for example a monoclonal antibody and a gamma-secretase inhibitor for use in treating cancer.

The inhibitors of γ-secretase complex (GSIs) represent a broad and heterogeneous family of molecules originally developed to counteract the production of amyloid-beta precursor protein (APP), the primary components of the amyloid plaque, in the brain of patients affected with the Alzheimer's disease [32]. However, given that Notch pathway represents one of the best characterized γ-secretase substrates and Notch-activating mutations are associated with several hematological malignancies and solid tumors, GSIs have been progressively tested as antitumor agents, alone or in combination with one or more conventional chemotherapeutic drugs [33].

In particular, PF-03084014, also known as nirogacestat, is a tetralin imidazole γ-secretase inhibitor originally developed by Pfizer Inc. to reduce amyloid-β (Aβ) production in patients affected with Alzheimer's disease [34,35], and subsequently tested as stand-alone chemotherapeutic agent or in combination with conventional anti-cancer drugs in the context of several clinical and preclinical models of hematological malignancies and solid tumors hallmarked by dysregulated Notch signaling pathway, such as desmoid tumors [36]. In particular, PF-03084014 entered late-stage clinical development (Phase II and Phase III trials) for the treatment of progressive and surgically unresectable desmoid tumors in paediatric (Phase II, NCT04195399) and adult patients (Phase III, NCT03785964) [37,38]. A report describing PF-03084014 safety and efficacy in adult patients has been recently published [39].

As stated above, according to the present invention two gamma-secretase inhibitors (GSIs), *i.e.* DAPT and PF-03084014 (nirogacestat) showed promising results in counteracting fibrotic behavior of recessive dystrophic epidermolysis bullosa fibroblasts.

In particular, according to the experimental results reported in Example 1, the fibrogenic properties of Notch signaling cascade in RDEB-associated fibrosis were shown. In particular, it was confirmed that the JAG1/NOTCH1 axis is over-expressed/over-activated in primary RDEB fibroblasts (RDEB-FBs) as compared to control cells [24]. In addition, according to the experimental results reported below, it was demonstrated for the first time by *in vitro* assays (*i.e.* functional analyses in JAG1-silenced RDEB-FBs) that the JAG1/NOTCH1 axis has a functional involvement in different fibrotic processes, including the cell ability to contract type I collagen lattices and the expression of typical myofibroblasts markers.

In addition, as stated above, according to the present invention it has been found that Notch pathway inhibition by two different gamma-secretase inhibitors (GSIs), *i.e.* DAPT and PF-03084014 (nirogacestat), attenuated a variety of fibrotic behaviors hallmarking RDEB-FBs. In particular, as reported in the experimental results shown below in Example 1, RDEB-FBs treated with DAPT and PF-03084014 showed:
(i) a significant reduction of their contractile ability and migration rate;
(ii) a diminished collagen deposition in the extracellular matrix; and
(iii) a down-regulation of the expression levels of several contractile/fibrotic proteins, including α-SMA, CNN1, TAGLN and JAG1.

In addition, according to the experimental data shown below, both DAPT- and PF-03084014-treated RDEB-FBs exhibited an increased ability to degrade a layer of type I collagen, suggesting that GSI can ameliorate the balance between collagen type I synthesis and degradation in cells endowed with fibrotic traits. In most functional assays and expression analyses the ability of DAPT and PF-03084014 to counteract the aforementioned fibrogenic processes and markers was maintained in RDEB-FBs treated with TGF-β1, highlighting their anti-fibrotic activity even in a challenging context. Finally, RDEB-FBs treated with 20 µM of PF-03084014 for 48 h showed a significant reduction of TGF-β1 secreted in the culture medium and a diminished proliferation rate.

Therefore, on the basis of the above-mentioned experimental results of Example 1, according to the present invention inhibitors of γ-secretase complex, in particular PF-03084014 and DAPT, can be advantageously used for the treatment of inherited epidermolysis bullosa, in particular for recessive dystrophic epidermolysis bullosa and its associated fibrosis.

According to the experimental results reported below in Example 1, PF-03084014 showed a greater anti-fibrotic activity as compared to DAPT. This proved true in all functional assays executed, in particular in the analysis of TGF-β1 secreted in the culture medium, and in the analysis of the expression levels of different pro-fibrotic markers. In addition, primary RDEB fibroblasts treated for 24 h with low doses of PF-03084014 (*i.e.* 0.1, 0.5 and 1 µM) exhibited a huge reduction in the amount of the intranuclear, biologically active form of NOTCH1 receptor (N1-ICD) as compared to cells treated with DAPT at the same dosages and time point. On the other hand, PF-03084014 and DAPT showed a comparable impact on the intranuclear levels of N1-ICD, when the two compounds were administered to cells at 5, 10 and 20 µM for 24 h.

Therefore, according to the present invention, PF-03084014 could be advantageously used as innovative drug to counteract RDEB-associated fibrosis, on the basis of the promising results shown below in Example 1 coupled with the existing know-how derived from preclinical and clinical studies (already completed or currently ongoing) aimed at evaluating dose range and therapeutic windows, adverse effects, molecular targets and antitumor efficacy of PF-03084014 in a variety of cancer models.

It is therefore specific object of the present invention a Gamma-secretase inhibitor for use in the treatment of inherited epidermolysis bullosa and/or a fibrosis associated to inherited epidermolysis bullosa, wherein said gamma-secretase inhibitor inhibits the multiprotein complex of gamma-secretase through a direct mechanism.

For Gamma-secretase inhibitor (or γ-secretase inhibitor or GSI) is intended a molecule able to inhibit the proteolytic activity of the gamma-secretase complex, which is a multi-subunit protease complex that cleaves a variety of single-pass transmembrane proteins, including Notch receptors, at specific residues within their transmembrane domains.

In particular, a gamma-secretase inhibitor according to the invention can be a TSA, *i.e.* a transition state analog, or a non-TSA, based on the mechanism of action.

According to the present invention, said gamma-secretase inhibitor inhibits the multiprotein complex of gamma-secretase through a direct mechanism, more in particular through the formation of chemical bonds between said inhibitor and specific amino acid residues of the component proteins of the multiprotein complex of gamma-secretase, for example Presenilin 1.

The inhibiting binding of the gamma-secretase inhibitor to the component proteins of the gamma-secretase complex can cause different effects, including but not limited to a conformational change of specific functional sites of the proteins belonging to the gamma-secretase complex, an interference with the recruitment of the effective biological substrates or a greater binding affinity of the inhibitor with the proteolytic complex of gamma-secretases compared to the affinity that the protein(s) of the complex would have with their biological substrates.

According to an embodiment of the present invention, when said Gamma-secretase inhibitor is for use in the treatment of inherited epidermolysis bullosa, said gamma-secretase inhibitor is not DAPT ((N-[N-(3,5-difluorophenacetyl)-L-alanyl]-S-phenylglycine t-butyl ester)) and is not DBZ (dibenzazepine).

According to the present invention, the inherited epidermolysis bullosa can be chosen from the group consisting of recessive dystrophic epidermolysis bullosa, dominant dystrophic epidermolysis bullosa (DDEB) and junctional epidermolysis bullosa (JEB), preferably recessive dystrophic epidermolysis bullosa.

According to the present invention, the fibrosis can be recessive dystrophic epidermolysis bullosa-associated fibrosis.

According to the present invention, said Gamma-secretase inhibitor can be chosen from the group consisting of Nirogacestat (also named PF-03084014, having chemical formula [(S)-2-((S)-5,7-difluoro-1,2,3,4-tetrahydronaphthalen-3-ylamino)-N-(1-(2-methyl-1-(neopentylamino)propan-2-yl)-1H-imidazol-4-yl)pentanamide]), DAPT (also named GSI-IX, having chemical formula (N-[N-(3,5-difluorophenacetyl)-L-alanyl]-S-phenylglycine t-butyl ester), RO492909, Semagacestat (also named LY-450139), BMS-906024 (also named AL101), Avagacestat (also named BMS-70816), MK-0752, preferably Nirogacestat.

The present invention concerns also a pharmaceutical composition comprising a gamma-secretase inhibitor, together with one or more pharmaceutically acceptable excipients and/or adjuvants, for use in the treatment of inherited epidermolysis bullosa and/or a fibrosis associated to inherited epidermolysis bullosa, wherein said gamma-secretase inhibitor inhibits the multiprotein complex of gamma-secretase through a direct mechanism.

According to the present invention, the inherited epidermolysis bullosa for which the pharmaceutical composition of the invention is used can be chosen from the group consisting of recessive dystrophic epidermolysis bullosa, dominant dystrophic epidermolysis bullosa (DDEB) and junctional epidermolysis bullosa (JEB), preferably recessive dystrophic epidermolysis bullosa.

According to the present invention, the fibrosis for which the pharmaceutical composition of the invention is used can be recessive dystrophic epidermolysis bullosa-associated fibrosis.

According to the present invention, the Gamma-secretase inhibitor of said pharmaceutical composition can be chosen from the group consisting of Nirogacestat (also named PF-03084014, having chemical formula [(S)-2-((S)-5,7-difluoro-1,2,3,4-tetrahydronaphthalen-3-ylamino)-N-(1-(2-methyl-1-(neopentylamino)propan-2-yl)-1H-imidazol-4-yl)pentanamide]), DAPT (also named GSI-IX, having chemical formula (N-[N-(3,5-difluorophenacetyl)-L-alanyl]-S-phenylglycine t-butyl ester), RO492909, Semagacestat (also named LY-450139), BMS-906024 (also named AL101), Avagacestat (also named BMS-70816), MK-0752, preferably Nirogacestat.

According to the present invention, said pharmaceutical composition can comprise one or more Gamma-secretase inhibitors, for example it can comprise Nirogacestat together with DAPT.

According to the present invention, said pharmaceutical composition can further comprise a product chosen from the group consisting of anti-inflammatory drugs, losartan, Angiotensin (Ang)-(1-7) heptapeptide and Ang-(1-7)-based pharmaceutical products, such as TXA127 and oral formulations of the heptapeptide angiotensin-(1-7).

The present invention concerns also a combination of a gamma-secretase inhibitor with a product chosen from the group consisting of anti-inflammatory drugs, losartan, Angiotensin (Ang)-(1-7) heptapeptide, Ang-(1-7)-based pharmaceutical products, such as TXA127 and oral formulations of the heptapeptide angiotensin-(1-7), topical hydrogel formulations containing thymosin beta-4 (Tβ4), such as RGN-137, a topical gel formulation of the peptide Tβ4 for the treatment of chronic wounds, gels containing dry extract from birch bark for the topical treatment of partial thickness wounds, such as Filsuvez^{®} (also named AP101/Oleogel-S10), and topical gels containing Decorin, for separate or sequential use in the treatment of inherited epidermolysis bullosa and/or a fibrosis associated to inherited epidermolysis bullosa, wherein said gamma-secretase inhibitor inhibits the multiprotein complex of gamma-secretase through a direct mechanism.

"Separate use" is understood as meaning the administration, at the same time, of the compounds of the combination according to the invention in distinct pharmaceutical forms.

"Sequential use" is understood as meaning the successive administration of the compounds of the combination according to the invention, each in a distinct pharmaceutical form.

According to the present invention, the inherited epidermolysis bullosa for which said combination is used can be chosen from the group consisting of recessive dystrophic epidermolysis bullosa, dominant dystrophic epidermolysis bullosa (DDEB) and junctional epidermolysis bullosa (JEB), preferably recessive dystrophic epidermolysis bullosa.

According to the present invention, the fibrosis for which said combination is used can be recessive dystrophic epidermolysis bullosa-associated fibrosis.

According to the present invention the Gamma-secretase inhibitor of said combination can be chosen from the group consisting of Nirogacestat (also named PF-03084014, having chemical formula [(S)-2-((S)-5,7-difluoro-1,2,3,4-tetrahydronaphthalen-3-ylamino)-N-(1-(2-methyl-1-(neopentylamino)propan-2-yl)-1H-imidazol-4-yl)pentanamide]), DAPT (also named GSI-IX, having chemical formula (N-[N-(3,5-difluorophenacetyl)-L-alanyl]-S-phenylglycine t-butyl ester), RO492909, Semagacestat (also named LY-450139), BMS-906024 (also named AL101), Avagacestat (also named BMS-70816), MK-0752, preferably Nirogacestat.

On the basis of the Phase III clinical trial for the treatment of progressive desmoid tumors in adults (Phase III, NCT03785964) [39], it is plausible that PF-03084014 (Nirogacestat) can be administered in recessive dystrophic epidermolysis bullosa patients, according to the present invention, at 150 mg by mouth (*e.g.* oral tablets), twice daily, continuously, in 28-day cycles.

The present invention now will be described by an illustrative, but not limitative way, according to preferred embodiments thereof, with particular reference to the example and the enclosed drawings, wherein:

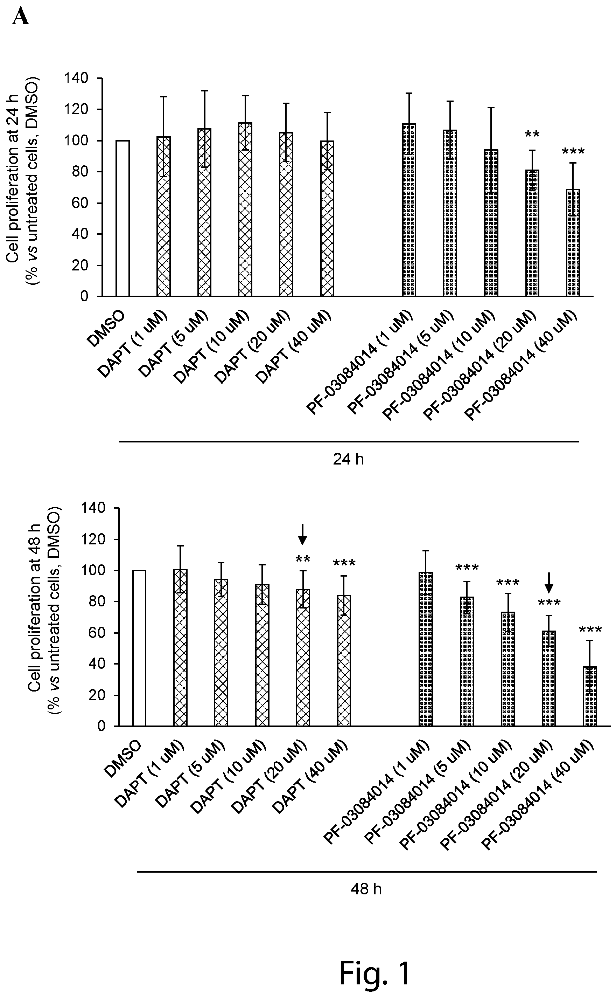

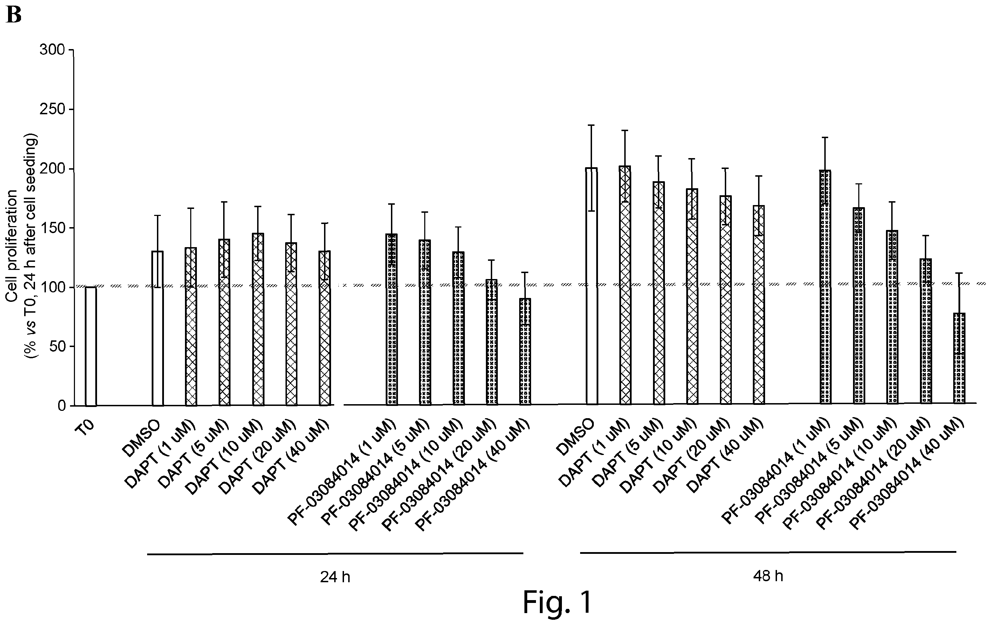

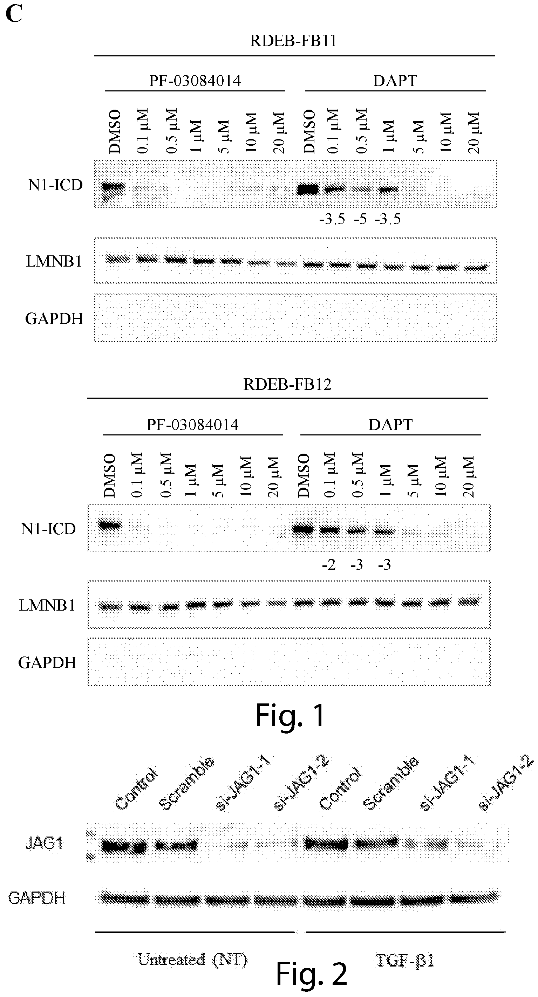

- **Figure 1** shows **the capability of PF-03084014 (i) to induce a time- and dose-dependent reduction of cell proliferation in primary fibroblasts from healthy subjects and (ii) to decrease the protein levels of the cleaved and biologically active form of NOTCH 1 receptor into the nucleus of primary fibroblasts from RDEB patients.** 3-(4,5-dimethyl-2-thiazolyl)-2,5-diphenyl-2H-tetrazolium bromide (MTT) assay showing the impact of DAPT and PF-03084014 treatment on viability and proliferation rate of three primary fibroblast strains from healthy controls (NH-FBs). Absorbance values (Abs) were converted in percentage by using the following formula: Abs _{DAPT or PF-03084014} x 100/ Abs _{REFERENCE WELLS}. Data are presented as mean ± standard deviation (SD) of at least eight technical replicates per condition. Cells treated with the vehicle DMSO (Panel A), and cells analyzed 24 h after seeding (T0) (Panel B) were considered as reference wells. NH-FBs were growth in DMEM medium containing 10% fetal bovine serum (FBS). (**Panel A**) NH-FBs treated for 48 h with 20 and 40 µM of DAPT showed a slight but statistically significant reduction of proliferation rate. Conversely, the effects of 20 and 40 µM of PF-03084014 were observed after 24 h of treatment, and became more evident after 48 h of treatment, even at low doses (5 and 10 µM). (**Panel B**) NH-FBs treated with PF-03084014 at 40 µM showed a reduction of cell viability as compared to cells before GSI treatment (T0) (dotted line). On the other hand, it was shown that 5-20 µM of PF-03084014 affected fibroblast proliferation in a time- and dose-dependent manner, in the absence of overt cytotoxicity. Based on the results, it was chosen to use GSIs at 20 µM (black arrows) as it was the minimal drug concentration inducing a reduction of cell proliferation after 24 h of treatment. (**Panel C**) Immunoblotting analysis showing the protein levels of the cleaved and biologically active form of NOTCH1 receptor (N1-ICD, NOTCH1 intracellular domain) in nuclear extracts of primary fibroblasts from RDEB patients (RDEB-FBs, n=2) treated for 24 h with DAPT and PF-03084014 at different doses, ranging from 0.1 to 20 µM. Cells treated with the vehicle dimethyl sulfoxide (DMSO) were used as control. The reduced nuclear localization of N1-ICD indicates Notch pathway inhibition. This head-to-head comparison experiment between DAPT and PF-03084014 highlights a greater ability of PF-03084014 in inhibiting NOTCH1 cleavage and its nuclear translocation at lower doses (*i.e.* 0.1, 0.5, 1 µM) as compared to DAPT-treated cells. Lamin B1 (LMNB1) and glyceraldehyde-3-phosphate dehydrogenase (GAPDH) served as loading controls for the nuclear and cytoplasmic protein fractions, respectively. ** P-value <0.01, *** P <0.001.
- **Figure 2** shows **protein levels of Jagged 1 (JAG1) in primary fibroblasts from RDEB patients transfected with two different short interfering RNAs (si-JAG1-1 and si-JAG1-2) targeting JAG1 mRNA.** Immunoblotting analysis showing JAG1 protein levels in primary RDEB fibroblasts (RDEB-FBs) transfected with two short interfering RNAs (siRNAs) targeting JAG1 mRNA (*i.e.* siJAG1-1 and siJAG1-2). At 24 h from JAG1 silencing, RDEB-FBs were treated with TGF-β1 at 2 ng/mL for additional 24 h.

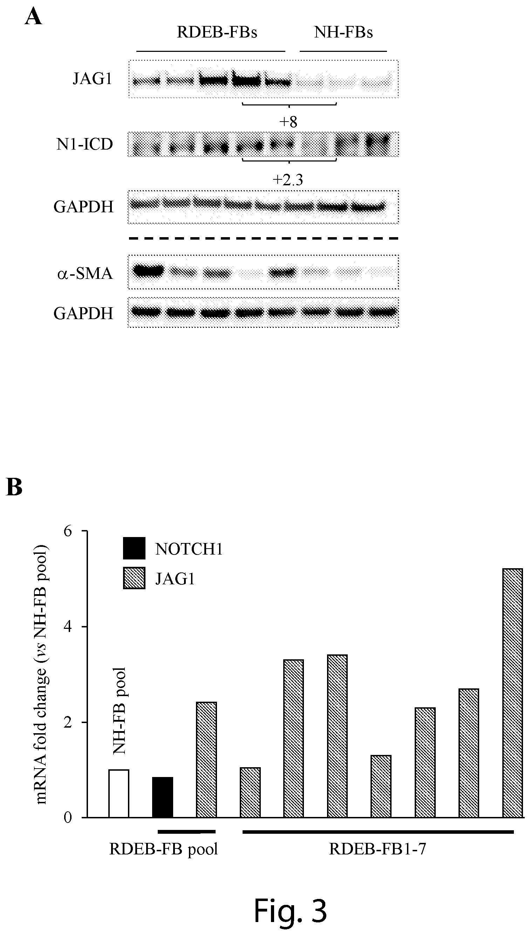

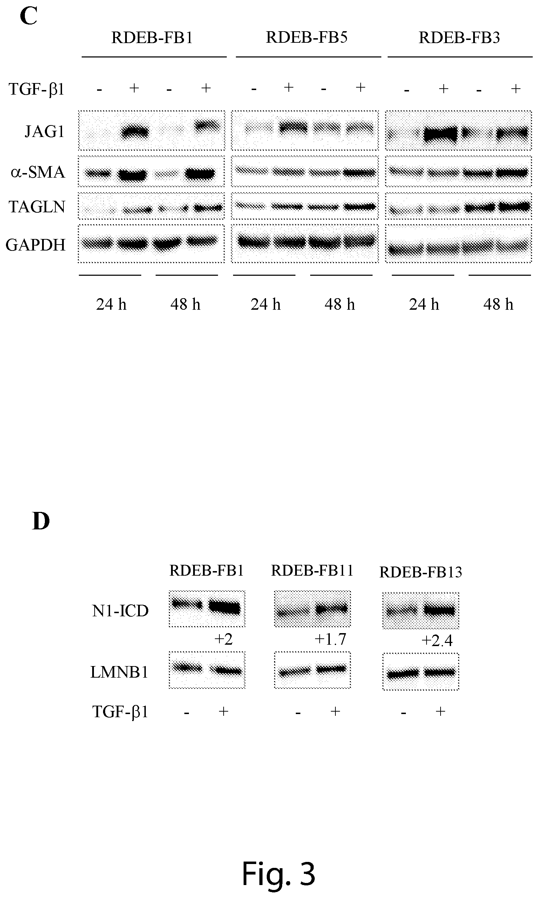

- **Figure 3** shows that **Jagged 1 and the cleaved product of NOTCH1 receptor are over-expressed in primary skin fibroblasts from RDEB patients and activated by transforming growth factor-β1. (A)** Immunoblotting (IB) analysis showing protein levels of the canonical Notch ligand Jagged 1 (JAG1), the cleaved product of the Notch receptor NOTCH1 (NOTCH1 intracellular domain, N1-ICD), and the fibrotic marker alpha-smooth muscle actin (α-SMA) in primary fibroblasts from RDEB patients (RDEB-FBs) (n=5) with respect to fibroblasts from healthy subjects (NH-FBs) (n=3). N1-ICD represents the ultimate, biologically active cleavage product of Notch receptor processing. Glyceraldehyde-3-phosphate dehydrogenase (GAPDH) was used as loading control. Dotted lines separate distinct IB experiments. **(B)** Real time-PCR analysis of JAG1 and NOTCH1 transcripts in RDEB-FBs (n=7) and NH-FBs (n=5). Note the increase of JAG1 mRNA expression levels in the majority of RDEB-FB strains analyzed (RDEB-FB1-7). GAPDH was used as housekeeping gene. **(C)** IB analysis showing the protein levels of the fibrosis markers JAG1, α-SMA and transgelin (TAGLN) in RDEB-FBs (n=3) treated with the prototypical pro-fibrotic cytokine transforming growth factor-β1 (TGF-β1) at 2 ng/mL for 24 and 48 h. GAPDH was used as loading control. α-SMA and TAGLN up-regulation in response to TGF-β1 treatment served as positive control to prove the effectiveness of TGF-β1 stimulation.**(D)** IB analysis showing the protein levels of the cleaved and biologically active form of NOTCH1 receptor (N1-ICD, NOTCH1 intracellular domain) in nuclear extracts of RDEB-FBs (n=3) treated with 2 ng/mL of TGF-β1 for 6 h. Lamin B1 (LMNB1) served as loading control for nuclear protein lysates.

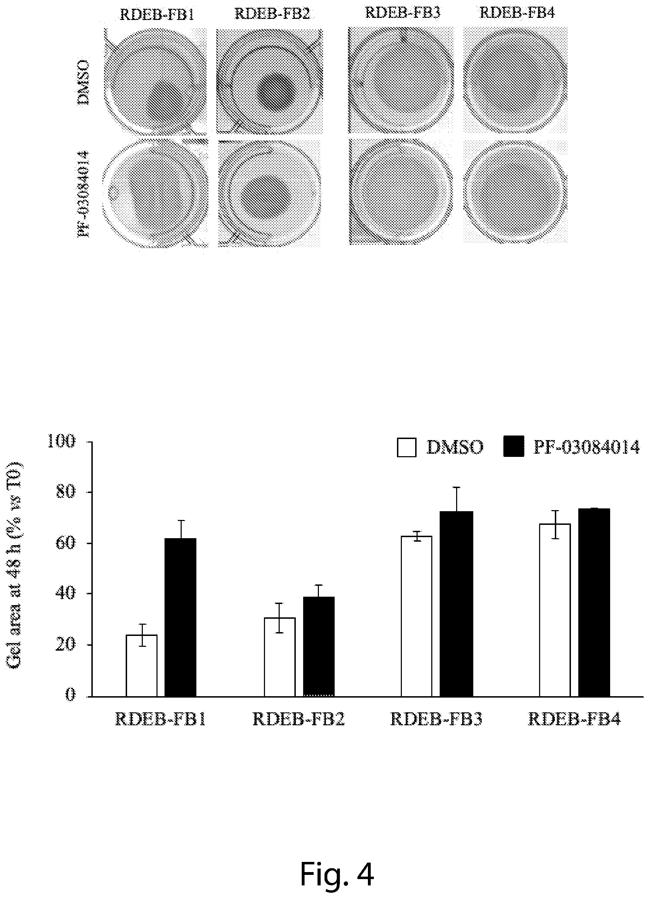

- **Figure 4** shows the **effect of the gamma-secretase inhibitor PF-03084014 on the contractile ability of primary fibroblasts from RDEB patients, in the absence of TGF-β1 as contraction booster. (Upper panel)** Representative images showing collagen lattice contraction (CLC) assay on primary fibroblasts from RDEB patients (RDEB-FBs) treated with the gamma-secretase inhibitor PF-03084014, in the absence of transforming growth factor-β1 (TGF-β1) as inducer of contraction. Experiments were performed in triplicate. (**Lower panel)** Histogram showing the percentage of contraction with respect to the initial gel areas, i.e. collagen lattices measured before stimulation with PF-03084014 (time 0, T0). Lower values indicate an increased capability to contract the collagen matrix.

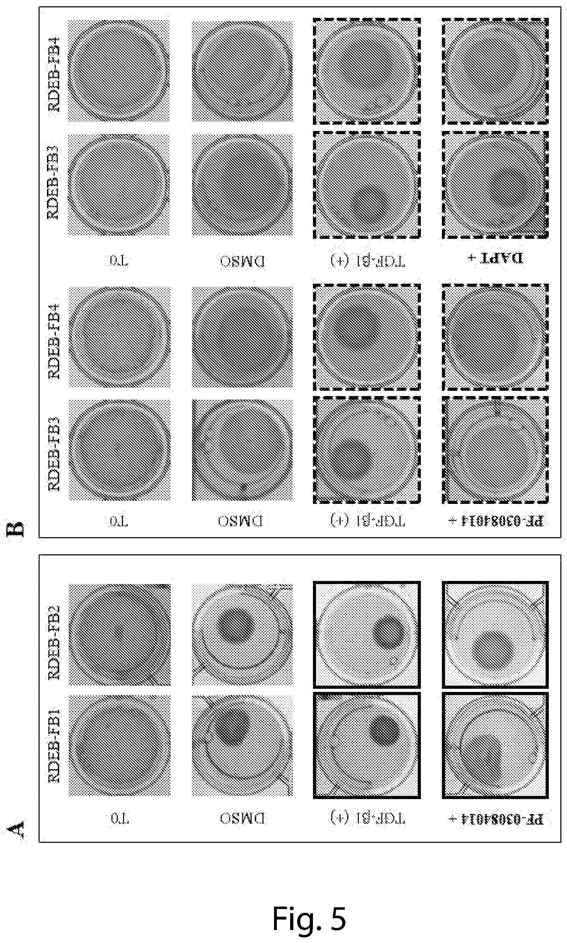

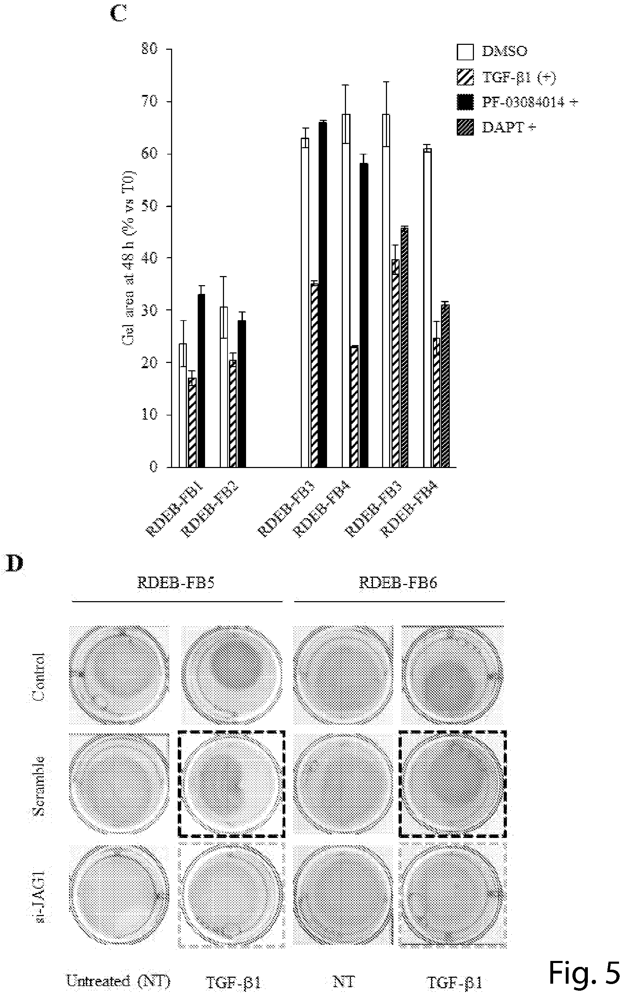

- **Figure 5** shows that **pharmacological and siRNA-mediated inhibition of Notch pathway impairs contractile capability of primary fibroblasts from RDEB patients. (A-B)** Collagen lattice contraction (CLC) assay of primary fibroblasts from RDEB patients (RDEB-FBs) treated for 48 h with the gamma-secretase inhibitors (GSIs) DAPT and PF-03084014 in the presence of TGF-β1 as contraction booster. Cells treated with the vehicle dimethyl sulfoxide (DMSO) were used as control. **(C)** Histograms showing the percentage of contraction with respect to the initial gel areas, *i.e.* collagen lattices measured at the detachment of collagen gels from the wells, and before the treatment with GSIs and TGF-β1 (time 0, T0). Lower values indicate an increased cell capability to contract the collagen matrix, and thus a major activation of pro-fibrotic processes. **(D)** CLC assay on two RDEB-FB strains transiently transfected with a short interfering RNA (siRNA) targeting Jagged 1 (si-JAG1) and a non-targeting scrambled control molecule (Scramble), with and without TGF-β1 stimulation. Untransfected cells are labelled as "control". Note that the capability of JAG1-silenced RDEB-FBs to contract the surrounding type I collagen matrix was particularly reduced in the presence of TGF-β1 as contraction booster.

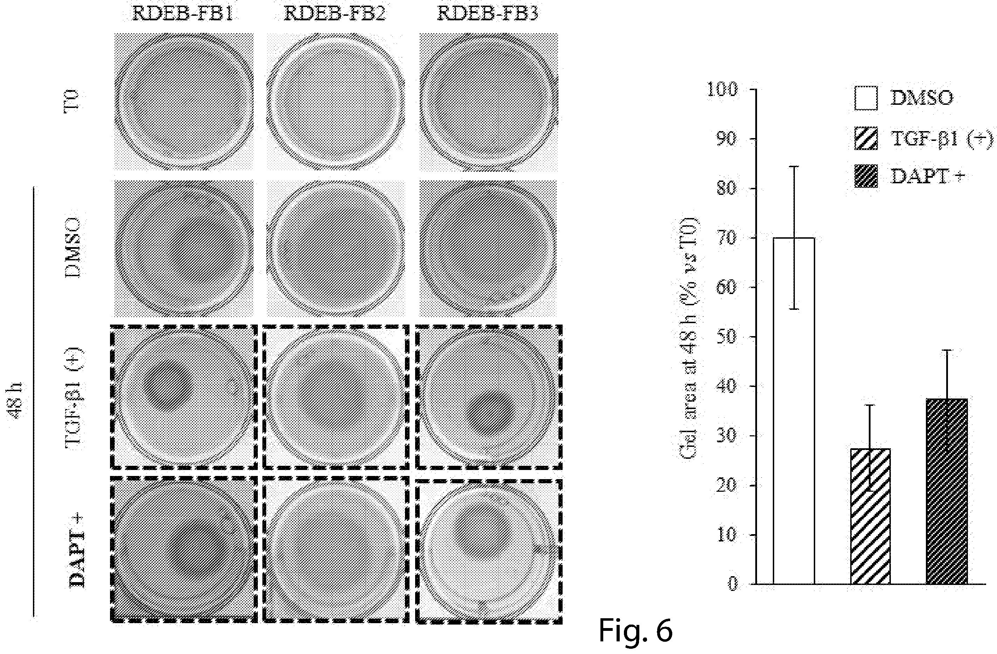

- **Figure 6** shows that **the gamma-secretase inhibitor DAPT lessens the contractile ability of primary fibroblasts from RDEB patients. (Left panel)** Collagen lattice contraction (CLC) assay on primary fibroblasts from RDEB patients (RDEB-FBs) (n=3) treated with the gamma-secretase inhibitor DAPT, in the presence of transforming growth factor-β1 (TGF-β1) as inducer of contraction. Experiments were performed in triplicate. (**Right panel)** Histogram showing the percentage of contraction with respect to the initial gel areas, *i.e.* collagen lattices measured before stimulation with DAPT and TGF-β1 (time 0, T0). Lower values indicate an increased capability to contract the collagen matrix.

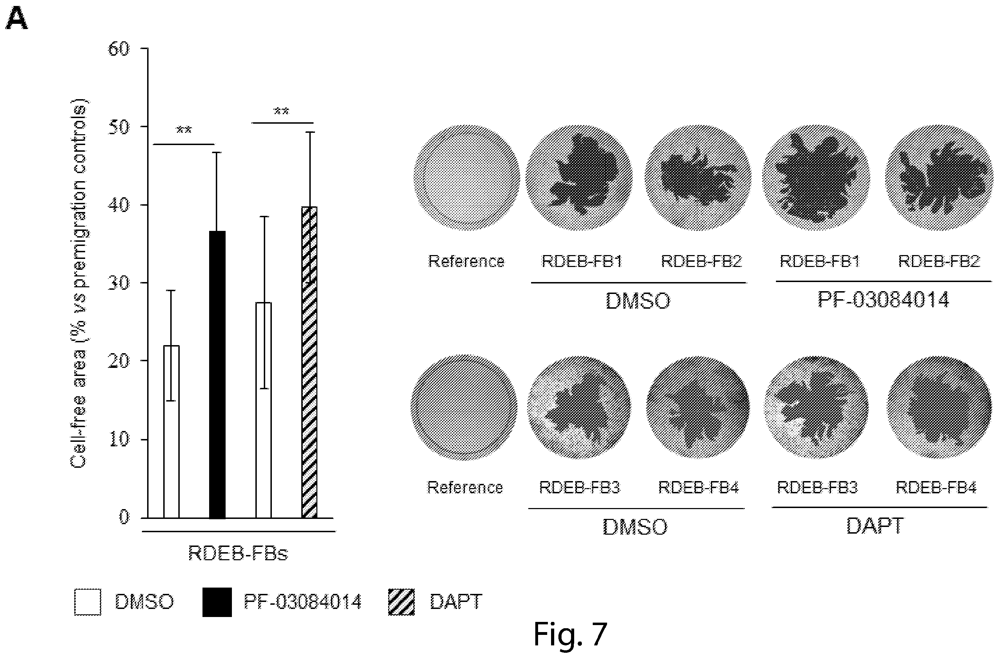

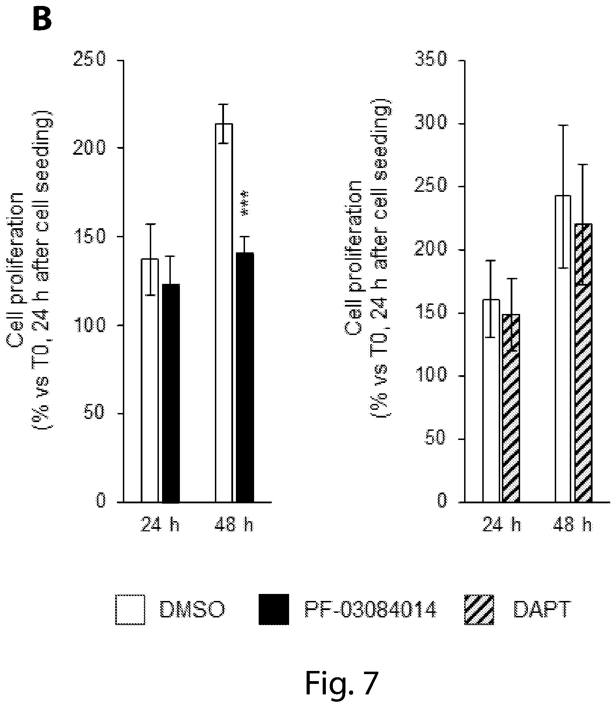

- **Figure 7** shows that **pharmacological inhibition of Notch pathway reduces migratory ability and proliferative rate of primary fibroblasts from RDEB patients. (A)** Cell migration assay of primary fibroblasts from RDEB patients (RDEB-FBs) treated with PF-03084014, DAPT or the vehicle DMSO for 24 h. The histogram shows the percentage of cell-free area with respect to reference (premigration controls). Data are presented as mean ± standard deviation (SD) of at least three replicates (n = 3). Smaller cell-free areas indicate an increased cell ability to migrate. **(B)** 3-(4,5-dimethyl-2-thiazolyl)-2,5-diphenyl-2H-tetrazolium bromide (MTT) proliferation assay of RDEB-FBs treated or not with PF-03084014 and DAPT. The histogram represents the percentage of proliferation with respect to reference, *i.e.* cells assayed before drug treatments (time 0, T0). Data represent mean ± SD of at least six replicates (n = 3). ** P-value <0.01, *** P <0.001.

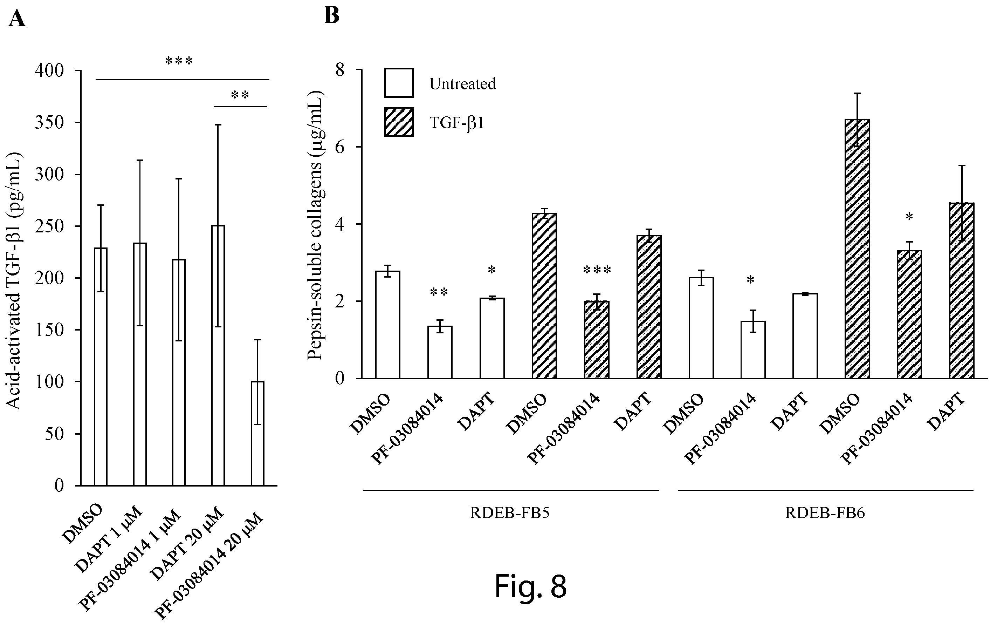

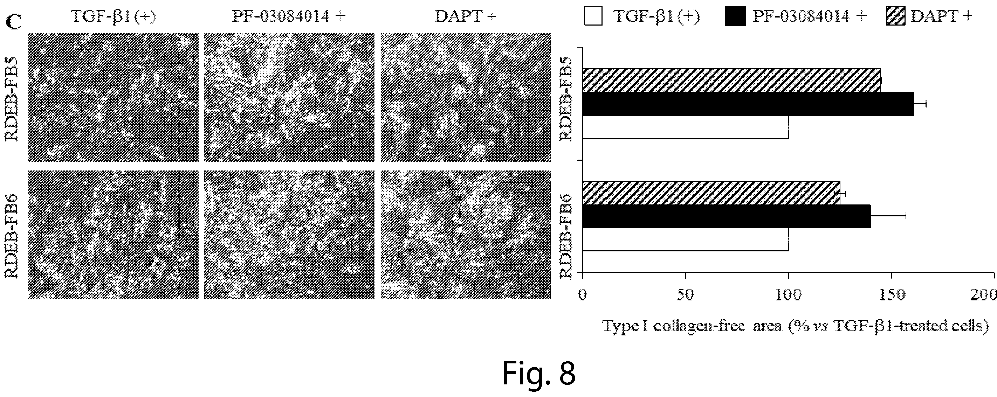

- **Figure 8** shows that **the pharmacological inhibition of Notch pathway reduces TGF-β1 secretion and collagen deposition into the extracellular matrix by primary fibroblasts from RDEB patients, and increases the cell capability to degrade a layer of type I collagen fibrils. (A)** The amount of TGF-β1 secreted by primary fibroblasts from RDEB patients (RDEB-FBs) in the presence of DAPT and PF-03084014 was assessed by ELISA assay. The histogram shows the amount of acid-activated TGF-β1 (pg/mL) in conditioned media of three RDEB-FB strains treated or not with 1 or 20 µM of DAPT and PF-03084014 for 48 h. Cells treated with the vehicle dimethyl sulfoxide (DMSO) were used as control. Data are presented as mean ± standard deviation (SD) of TGF-β1 concentration obtained for each experimental conditions in three different RDEB-FB strains. **(B)** Histogram showing the amount of pepsin-soluble collagens (µg/mL) deposited into the extracellular matrix (ECM) by RDEB-FBs treated for 96 h with PF-03084014 (5 µM) and DAPT (20 µM), in the presence or in the absence of TGF-β1 as trigger of collagen synthesis. DMSO-treated cells were used as controls. Collagen amount was measured by employing the Sirius red staining-based Sircol assay (Biocolor, Carrickfergus, Northern Ireland - UK). Data represent mean ± SD of two replicates. **(C, Left panel)** Representative images showing the collagenolytic activity of two RDEB-FB strains (RDEB-FB5 and RDEB-FB6) seeded on a reconstituted type I collagen film, and treated for 96 h with the GSIs PF-03084014 (5 µM) and DAPT (20 µM), in the presence of TGF-β1 (+). Collagen degradation is detected as white areas on a dark grey background. **(C, Right panel)** Histogram showing the percentage of collagen degradation in GSI-treated cells with respect to unstimulated controls. Experiments were performed in duplicate. * P-value <0.05, ** P-value <0.01, *** P <0.001.

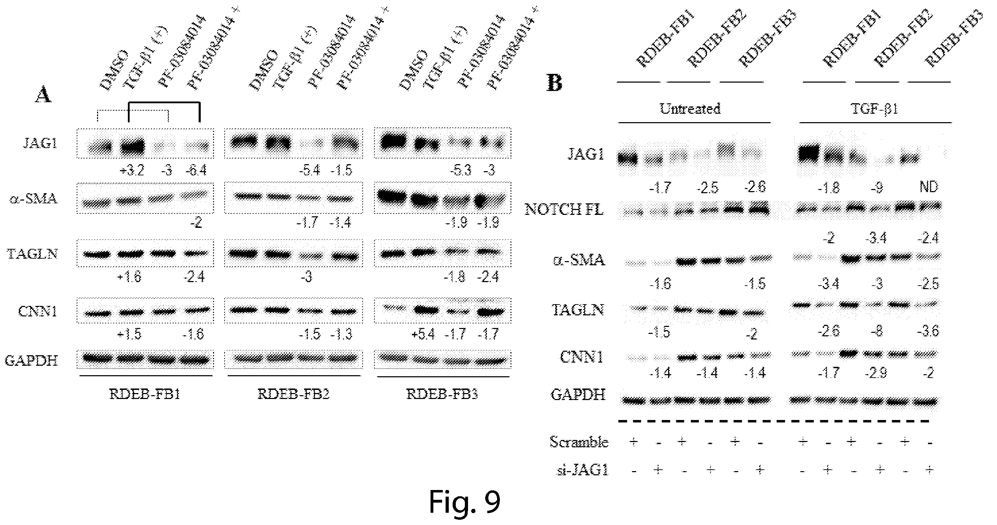

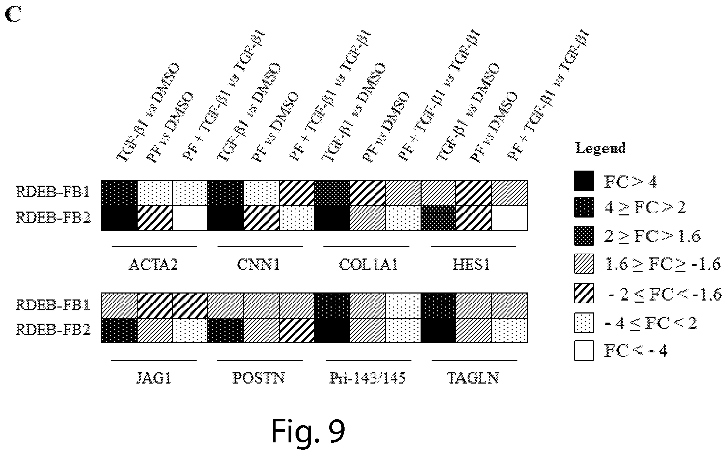

- **Figure 9** shows that **pharmacological and siRNA-mediated inhibition of Notch pathway down-regulates myofibroblast markers. (A)** Immunoblotting (IB) analysis showing the protein levels of the fibrotic markers alpha-smooth muscle actin (α-SMA), transgelin (TAGLN), calponin (CNN1) in primary fibroblasts from RDEB patients (RDEB-FBs) (n=3) treated for 24 h with the gamma-secretase inhibitor PF-03084014 (PF) (20 µM), with or without TGF-β1 stimulation. DMSO-treated cells were used as controls. **(B)** IB analysis showing the protein levels of the fibrotic markers α-SMA, TAGLN, CNN1 and the unprocessed NOTCH1 receptor (NOTCH FL, Notch Full Length) in JAG1-silenced RDEB-FBs (n=3), treated or not with TGF-β1. Glyceraldehyde-3-phosphate dehydrogenase (GAPDH) was used as loading control in immunoblotting analyses. **(C)** Real-time PCR analysis showing the expression levels of a selection of contractile/fibrotic markers, including α-SMA, CNN1, TAGLN and JAG1, in RDEB-FBs treated with PF-03084014 (PF) both in basal culture conditions and in the presence of TGF-β1. HES1 (hes family bHLH transcription factor 1) is a transcriptional target of Notch. Periostin (POSTN) and Pri-miR-143/145 represent two validated pro-fibrotic genes in primary RDEB fibroblasts [24,40]. Hypoxanthine phosphoribosyltransferase 1 (HPRT1) was used as housekeeping gene.

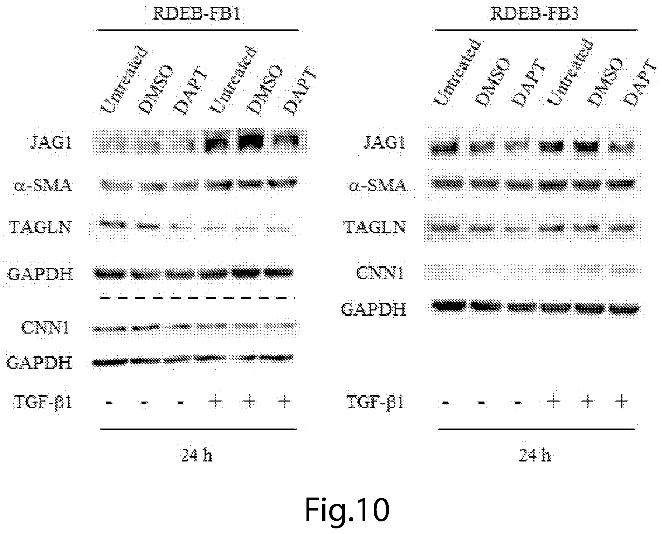

- **Figure 10** shows **protein levels of a selection of myofibroblast markers in primary fibroblasts from RDEB patients treated with the gamma-secretase inhibitor DAPT.** Immunoblotting (IB) analysis showing the protein levels of the fibrotic markers alpha-smooth muscle actin (α-SMA), transgelin (TAGLN), calponin (CNN1) in primary fibroblasts from RDEB patients (RDEB-FBs) (n=3) treated for 24 h with the gamma-secretase inhibitor DAPT, in the presence or in the absence of TGF-β1 as inducer of pro-fibrotic proteins. DMSO-treated cells were used as controls.

### EXAMPLE 1: Study of the fibrogenic properties of Notch signaling cascade in RDEB-associated fibrosis and of the inhibition of Notch pathway by gamma-secretase inhibitors (GSIs) according to the present invention.

### Material and Methods

### Cell cultures

Primary dermal fibroblasts from RDEB patients (RDEB-FBs) and healthy subjects (NH-FBs) were obtained from biopsies taken for diagnostic purposes or plastic surgery, respectively. *Informed consent was obtained prior to skin biopsy from patients*/*healthy subjects or their legal guardians, according to the current Italian legislation. This study was approved by the Ethical Committee of Bambino Gesù Children's Hospital, IRCCS, Rome, Italy* (*2470_OPBG_2021) and IDI-IRCCS, Rome, Italy (ID #660*/*1, 2021).*

Fibroblasts (passages 4-8) were cultured in Dulbecco's modified Eagle's medium (DMEM) supplemented with 10% fetal bovine serum (FBS), 50 U/mL of penicillin G, 50 µg/mL of streptomycin and 4 mM L-glutamine. For selected experiments, RDEB-FBs and NH-FBs were serum-starved overnight in serum-free medium containing 0.1% bovine serum albumin (BSA). TGF-β1 was used at 2 ng/mL (100-21C; PeproTech, London, UK).

### Pharmacological and short interfering RNA-mediated inhibition of Notch signaling

*Gamma-secretase inhibitors.* NH-FBs and RDEB-FBs were treated with two different gamma-secretase inhibitors (GSIs): DAPT (N-[N-(3,5-difluorophenacetyl)-L-alanyl]-S-phenylglycine t-butyl ester) (Sigma-Aldrich, St. Louis, MO, USA) and PF-03084014 [(S)-2-((S)-5,7-difluoro-1,2,3,4-tetrahydronaphthalen-3-ylamino)-N-(1-(2-methyl-1-(neopentylamino)propan-2-yl)-1H-imidazol-4-yl)pentanamide] (Selleck Chemicals LLC, Houston, TX, USA).

The chemical features of the gamma-secretase inhibitors DAPT and PF-03084014 (nirogacestat) are reported in Table 1, in particular chemical structure, IUPAC (International Union of Pure and Applied Chemistry) name, CAS (Chemical Abstract Service, USA) Registry Number (Cas. No), molecular formula and molecular weight of the gamma-secretase inhibitors DAPT (a) and PF-03084014 (b).

**Table 1**

| **a) DAPT (GSI-IX)** |
|---|
| 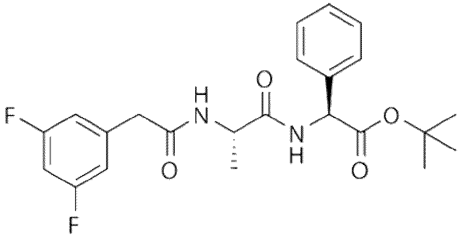 |
| *IUPAC name:* tert-butyl (2s)-2-[[(2s)-2-[[2(3,5difluorophenyl)acetyl]amino]propanoyl]amino]-2-phenylacetate |
| Cas. No: 208255-80-5 |
| *Molecular formula:* C₂₃H₂₆F₂N₂O₄ |
| *Molecular weight:* 432.46 |
| |

| **b) PF-03084014 (Nirogacestat)** |
|---|
| 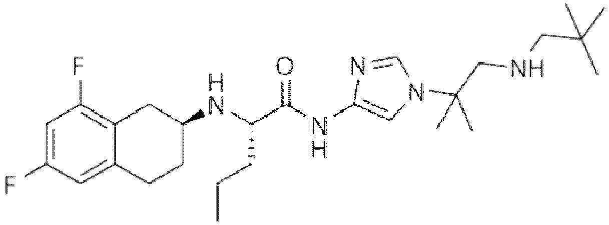 |
| *IUPAC name:* (2S)-2-[[(2*S*)-6,8-difluoro-1,2,3,4-tetrahydronaphtalen-2-yl]amino]-*N*-[1-[1-(2,2-dimethylpropylamino)-2-methylpropan-2-yl]imidazol-4-yl]pentanamide |
| Cas. No: 1290543-63-3 |
| *Molecular formula:* C₂₇H₄₁F₂N₅O |
| *Molecular weight:* 489.6 |

GSIs halt the processing of Notch receptor, and, in turn, play a role as indirect inhibitors of Notch signaling cascade. Following their reconstitution at 10 mM in dimethyl sulfoxide (DMSO), GSI aliquots were stored at -20°C until use, avoiding freeze-thaw cycles. GSI treatment was performed in DMEM containing 10% FBS, unless otherwise specified. According to previous reports, RDEB-FBs were serum-starved overnight in DMEM containing BSA prior to be treated with GSIs [31]. Cells treated with the vehicle DMSO were used as controls. GSIs were used at different concentrations ranging from 1 to 20 µM depending on the assay. GSI concentrations were selected according to a preliminary MTT viability test on three NH-FB strains (Figure 1 - Panel A and B) and the evaluation of the capability of DAPT and PF-03084014 at different doses (24 h of treatment) to reduce the production of the cleaved, biologically active product of NOTCH1 receptor (NOTCH1 intracellular domain, N1-ICD)(Figure 1 - Panel C).

*Transfection of short interfering RNAs targeting Jagged 1.* Two candidate short interfering RNA (siRNA) sequences complementary to different regions of the human JAG1 mRNA, si-JAG1-1 and si-JAG1-2, were chosen to be functionally evaluated in the context of transfection experiments. siJAG1-1 and siJAG1-2 sequences are indicated below: 5'-GAAUGUGAGGCCAAACCUU[dT][dT]-3' (SEQ ID NO:1) (si-JAG1-1, cod. SASI_Hs01_00100441) and 5'-CCUGUAACAUAGCCCGAAA[dT][dT]-3' (SEQ ID NO:2) (si-JAG1-2, cod. SASI_Hs01_00100442). Both siRNAs were purchased from Merck (Merck, Darmstadt, Germany). Lipofectamine 2000 (Thermo Fisher Scientific, Waltham, MA, USA) was used to transfect siRNAs, following manufacturer's protocol. After 48 h, the silencing efficiency of the two candidate siRNAs was assessed by immunoblotting (IB) (Figure 2). IB analysis demonstrated that both molecules were able to reduce JAG1 expression levels in the absence of TGF-β1 stimulation (2 ng/mL for 24 h) (Figure 2) as compared to cells transfected with a control scramble molecule (Scramble). Conversely, in the presence of TGF-β1, si-JAG1-2-transfected cells showed a major reduction of JAG1 protein levels as compared to cells transfected with si-JAG1-1 (Figure 2). Accordingly, si-JAG1-2, throughout the text indicated as si-JAG1, was selected for all downstream silencing experiments. The MISSION^{®} siRNA Universal Negative Control #1 (Merck) was used as negative control for siRNA transfections.

### Collagen lattice contraction assay

RDEB-FBs (5 x 10⁵ cells) were mixed in a collagen buffer and seeded into 12-well plates as previously described [24]. After gelation, fibroblast-populated collagen lattices were detached from the wells and left floating. RDEB-FBs were treated with 20 µM of DAPT and PF-03804014 in DMEM containing 0.1% BSA, in the presence or absence of TGF-β1 at 2 ng/mL, as contraction booster. JAG1-silenced RDEB-FBs were embedded in collagen lattices 24 h after siRNA transfection. In both cases, images were acquired with ChemiDoc^{™} XRS+ System (Bio-Rad, Hercules, CA, USA) at the detachment of collagen lattices from the wells (time 0, T0) and after 48 h. Gel areas were measured by ImageJ software and photos at T0 were used as references.

### Proliferation assay

Proliferation rate was assessed by 3-(4,5-dimethyl-2-thiazolyl)-2,5-diphenyl-2H-tetrazolium bromide (MTT) assay, following the manufacturer's protocol. In brief, three different strains of RDEB-FBs were seeded at the density of 4 x 10³ cells into 96-well plates. The day after, RDEB-FBs growing in DMEM containing 10% FBS were treated with 20 µM DAPT or PF-03084014 for 24 h or 48 h. Cells treated with the vehicle DMSO were used as controls. At the selected time points, medium was replaced with MTT solution and plates were incubated in a dry incubator at 37°C for 3 h. Thereafter, cells were lysed in DMSO to dissolve formazan crystals and absorbance values were recorded at 560 nm with an Infinite F50 microplate reader (Tecan, Mannedorf, Switzerland). Values recorded 24 h after cell seeding, before GSI stimulation (T0) were used as reference.

The reduction of cell proliferation is well-established effect of GSIs on a variety of cell types [41]. For these reasons, the appropriate concentrations of GSIs to use in the present study were selected based on preliminary MTT analyses on three NH-FB strains treated for 24 and 48 h with PF-03084014 and DAPT at increasing concentration (1, 5, 10, 20 and 40 µM) (Figure 1 - panel A), as described above.

### Cell migration assay

Migration rate was evaluated using an Oris^{™} Cell Migration Assay Kit (Platypus Technologies, Madison, WI, USA) following the manufacturer's instructions. Briefly, cells were seeded at high density (5 × 10⁴ per well) onto 96-well plates containing medical-grade silicon stoppers that restrict cell seeding to an outer annular region of the well, and serum starved overnight. The day after, silicon stoppers were removed and cells were transfected. Analysis of cell migration into the detection zone was performed after Giemsa stain (Sigma-Aldrich). The detection zone was photographed with a Leica DMi8 microscope (Leica Microsystems, Wetzlar, Germany) at 0 h (premigration controls) and 24 h after stopper removal. ImageJ was used to calculate the percentage of cell-free area. The cell migration assay of RDEB-FBs and NH-FBs grown under basal conditions was performed following the same protocol. No mitomycin C treatment to block cell proliferation was performed as (i) serum starved and over confluent cells used in the assay undergo contact inhibition of proliferation and (ii) migration was evaluated at a short time point (24 h).

### ELISA assay

Human TGF-β1 Quantikine ELISA Kit (R&D Systems, Minneapolis, MN, USA) was used to measure the amount of TGF-β1 (pg/mL) in serum-free conditioned media of three RDEB-FB strains, treated or not with PF-03084014 and DAPT at 1 or 20 µM for 48 h. DMSO-treated cells were used as controls. TGF-β1 Quantikine ELISA Kit was performed as per manufacturer's instructions. Samples were assayed in duplicate for each experimental condition. In order to detect both the latent precursor and the active form without latency-associated peptide cell supernatants were subjected to acid activation prior to be analyzed [42,43]. Absorbance values were recorded at 455 nm with an Infinite F50 microplate reader (Tecan, Männedorf, Switzerland) and analyzed by employing the GraphPad Prism 8.2 version (GraphPad Software Inc., San Diego, CA, USA).

### Collagen deposition analysis

The amount of collagens deposited into the extracellular matrix (ECM) was measured by using the Sirius red staining-based Sircol assay (Biocolor, Carrickfergus, Northern Ireland - UK). Briefly, two RDEB-FB strains were seeded into 12-well plates (55.000 cells/well), and growth for two days until they reached about 80-90% confluence. Then, cells were treated for 96 h with PF-03084014 (5 µM), DAPT (20 µM) or the corresponding amount of vehicle DMSO in serum-free media. GSI stimulation was performed in the presence or in the absence of TGF-β1 (2 ng/mL) as inducer of collagen synthesis. In order to further improve collagen deposition, RDEB-FB cultures were daily supplemented with 50 µg/mL of L-ascorbic acid (Merck) [44] and 100 µg/mL of dextran sulfate 500 kDa (Merck) [45]. ECM collagens were solubilized by scraping culture wells with a solution of 0.5 M acid acetic containing pepsin at 0.1 mg/mL (Pepsin E.C. 3.4.23.1, product code: P7012, Merck) and incubated overnight at 4°C in agitation. The day after, ECM lysates were neutralized with 100 µL of Acid Neutralizing Reagent (Biocolor), and concentrated by overnight incubation in ice-water mix, in the presence of 200 µL of Isolation & Concentration Reagent (Biocolor). Sample absorbance values were measured by BioTek Synergy H1 microplate reader (Agilent, Santa Clara, CA, USA) at 560 nm and analyzed through the GEN5^{™} software v. 3.04 (Agilent).

### Collagen degradation assay

The ability of RDEB-FBs to degrade a thin film of type I collagen (COL1) was assessed as previously described [46]. Briefly, COL1 from calf hides (Symatese, Chaponost, France), was diluted at a concentration of 375 µg/ml in 13 mM hydrochloric acid. Diluted COL1 solution was rapidly mixed on ice with one quarter-volume of a neutralizing phosphate buffer (20 ml of 0.2 M NaH₂PO₄ pH 7.5, 20 ml 0.1 M NaOH, 4.15 ml 5 M NaCl) and gently dispensed into 24-well plates. COL1 gelation was obtained maintaining COL1-coated plates at 37°C for 2 h. COL1 gels were dehydrated overnight in a laminar flow hood, washed with sterile water and PBS containing Penicillin/Streptomycin. Then, a droplet of complete medium containing 12.500 RDEB-FBs was gently poured in the center of the well, and cells were allowed to adhere in a humidified chamber for 3 h. Cells were maintained in Optimem I Medium (Thermo Fisher Scientific) for 5 days, medium containing supplements was replaced after 2 days. At the end of the incubation period, RDEB-FBs were detached by trypsin/EDTA and counted to be sure to evaluate collagenolytic activity of the same number of cells per condition. The residual COL1 film was stained for 15 min with a solution of Coomassie Brilliant Blue R-250 Dye (Thermo Fisher Scientific), the excess of stain was removed with a destain solution (30% ethanol, 7.5% CH₃COOH). Images were acquired with a 10x objective.

### RNA extraction and real-time PCR analysis

Total was extracted by TRIzol (Thermo Fisher Scientific) by monolayer fibroblast cultures, following manufacturer's instructions. RNA was reverse transcribed and amplified by using Power SYBR Green RNA-to-Ct 1-Step Kit (Thermo Fisher Scientific) on QuantStudio^{™} 7 Pro Real-Time PCR System (Thermo Fisher Scientific). Primer pairs used in this study are listed in Table 2: ACTA2, actin alpha 2, smooth muscle; CNN1, calponin; COL1A1, collagen type I alpha 1 chain; GAPDH, glyceraldehyde-3-phosphate dehydrogenase; HES1, hes family bHLH transcription factor 1; JAG1, jagged canonical Notch ligand 1; NOTCH1, notch receptor 1; POSTN, periostin; TAGLN, transgelin.

**Table 2**

| **Primer name (cDNA)** | **Forward sequence (5'-3')** | **Reverse sequence (5'-3')** |
|---|---|---|
| **ACTA2** | GCGTGGCTATTCCTTCGTT A (SEQ ID NO:3) | GACTCCATCCCGATGAAGGAT (SEQ ID NO:4) |
| **CNN1** | GGCCCAGAAGTATGACCAC C (SEQ ID NO:5) | GATGAATTCGCAAAGAATGATG CC (SEQ ID NO:6) |
| **COL1A1** | GGCCAAGACGAAGACATC CC (SEQ ID NO:7) | CGTCATCGCACAACACCTTG (SEQ ID NO:8) |
| **GAPDH** | GAAGGTGAAGGTCGGAGT C (SEQ ID NO:9) | GAAGATGGTGATGGGATTTC (SEQ ID NO:10) |
| **HES1** | AGAAAGATAGCTCGCGGC ATT (SEQ ID NO:11) | TACTTCCCCAGCACACTTGG (SEQ ID NO:12) |
| **JAG1** | GTCTCAACGGGGGAACTTG T (SEQ ID NO:13) | GCGTGCTCAGCAATTTCACA (SEQ ID NO:14) |
| **NOTCH1** | GACAGCCTCAACGGGTACA A (SEQ ID NO:15) | CACACGTAGCCACTGGTCAT (SEQ ID NO:16) |
| **POSTN** | TGGAAGAGACGGTCACTTC ACA (SEQ ID NO:17) | GTGGTACTTCATAAGAGCTTCGG (SEQ ID NO:18) |
| **Pri-miR-143/145** | AACTCCAGCTGGTCCTTAG (SEQ ID NO:19) | TCTTGAACCCTCATCCTGT (SEQ ID NO:20) |
| **TAGLN** | AACCACCGGGGTGAGAGG (SEQ ID NO:21) | GGGGAAAGCTCCTTGGAAGT (SEQ ID NO:22) |
| **HPRT1** | TGACACTGGCAAAACAATGC A (SEQ ID NO:23) | GGTCCTTTTCACCAGCAAGCT (SEQ ID NO:24) |

Relative mRNA expression levels were measured by the 2-ΔΔCT method [47]. Hypoxanthine phosphoribosyltransferase 1 (HPRT1) was used as housekeeping gene to normalize mRNA expression levels.

### Cytoplasmic and nuclear fractionation

RDEB-FBs were seeded on 100 mm cell culture dishes (8×10⁵ cells/dish). Subconfluent cells were treated with different concentrations of PF-03084014 or DAPT for 24 h (Fig. 1 - Panel C) or with 2 ng/mL of TGF-β1 for 6 h (Fig. 3D). DMSO-treated cells were used as controls. After cell harvesting with trypsin-EDTA, cytoplasmic and nuclear lysates were obtained using NE-PER^{™} Nuclear and Cytoplasmic Extraction Reagents kit (Thermo Fisher Scientific), following the manufacturer's instructions with slight modifications. The extraction protocol was scaled on a cell pellet volume of 20 µL and the extraction buffers were supplemented with phosphatase- and protease inhibitors (Merck).

### Immunoblotting

Whole-cell lysates were obtained in radioimmunoprecipitation assay (RIPA) buffer (Sigma-Aldrich) supplemented with phosphatase- and protease inhibitors (Merck). Different amounts of protein lysates were run under reducing conditions by using 4-12% gradient precast gels (Thermo Fisher Scientific) or hand-casting polyacrylamide gels (4-8%), depending on the protein to be assayed. Amersham ^{™} Protran^{®} Premium nitrocellulose membranes (pore size 0.2 µm) (Merck) were immunoblotted with primary antibodies indicated in Table 3 and incubated with horseradish peroxidase-linked secondary antibodies.

Primary antibodies used in this study are listed in Table 3: α-SMA, alpha smooth muscle actin; CNN1, calponin; GAPDH, glyceraldehyde 3-phosphate dehydrogenase; JAG1, jagged canonical Notch ligand 1; LMNB1, lamin B1; NOTCH1, notch receptor 1; NOTCH1, cleaved (intracellular domain, ICD); TAGLN, transgelin. TBS-T stands for Tris Buffer Saline with 20% Tween 20.

**Table 3**

| **Primary antibody** | **Clone** | **Company** | **Dilution** |
|---|---|---|---|
| α-SMA | 1A4 | Merck | 1:1000 (TBS-T, 5% milk) |
| CNN1 | 836701 | R&D Systems (Bio-Techne) | 1 µg/mL (TBS-T, 5% milk) |
| GAPDH | 14C10 | Cell Signaling Technology | 1:1000 (TBS-T, 5% milk) |
| JAG1 | 28H8 | Cell Signaling Technology | 1:500 (TBS-T, 1% milk) |
| LMNB1 | pAb (code: ab16048) | Abcam | 1:1000 (TBS-T, 5% milk) |
| NOTCH1 | BTAN20 | Developmental Studies Hybridoma Bank | 1:500 (TBS-T, 1% milk) |
| NOTCH1, cleaved (ICD) | D3B8 | Cell Signaling Technology | 1:500 (TBS-T, 1% milk) |
| TAGLN | 859112 | R&D Systems (Bio-Techne) | 1:1000 (TBS-T, 5% milk) |

Detection was performed using Cytiva ECL^{™} Prime Western Blotting System (Merck) or SuperSignal West Femto (Thermo Fisher Scientific), depending on the protein to be assayed. Antibody against Notch1 (all forms; clone bTAN20) was obtained from the Developmental Studies Hybridoma Bank (DSHB) at the University of Iowa (DSHB, Iowa City, IA, USA). GAPDH (clone 14C10), Jagged1 (clone 28H8) and NOTCH1 intracellular domain (clone D3B8) were obtained from Cell Signaling (Cell Signaling Technology Inc., Beverly, MA, USA). Antibodies against TAGLN (clone #859112) and CNN1 (clone #836701) were from R&D Systems (R&D Systems, Inc. Minneapolis, MN, USA). Antibody against α-SMA (clone 1A4) was purchased from Sigma-Aldrich. Antibody against lamin B1 (LMNB1) was from Abcam (Cambridge, UK). All the antibodies were used in accordance with the manufacturer's instructions. Glyceraldehyde-3-phosphate dehydrogenase (GAPDH) and LMNB1 were used as loading control.

### Results

### JAG1 and intracellular NOTCH1 are over-expressed in primary skin fibroblasts from RDEB patients, and their expression and activation are enhanced by TGF-β1

The expression levels of JAG1 and the cleaved form of NOTCH1 were analyzed by immunoblotting (IB) and real-time PCR in RDEB-FBs and NH-FBs. IB analysis revealed a significant increase in JAG1 and N1-ICD protein levels in RDEB-FBs as compared to the NH-FBs pool (Fig. 3A). These results were in keeping with previous findings (see Fig. 7 in Ref. [21]). Real-time PCR confirmed a significant increase of JAG1 mRNA amount, though less marked than that observed at protein level, pointing to post-translational regulatory mechanisms boosting JAG1 mRNA translation (Fig 3B). On the other hand, NOTCH1 mRNA levels were comparable between RDEB-FBs and NH-FBs (Fig. 3B). In keeping with the fibrogenic role of the JAG1/NOTCH1 axis, RDEB-FBs treated with TGF-β1 showed a further upregulation of JAG1 protein levels, which was maximal at 24 h (Fig. 3C), and an increased amount of the cleaved and biologically active form of NOTCH1 receptor (N1-ICD) in nuclear extracts after 6 h of stimulation (Fig. 3D).

### Pharmacological and short interference RNA-based inhibition of Notch signaling cascade impairs contractile capability of primary RDEB fibroblasts

Contractile ability is a typical feature of myofibroblasts, and its evaluation through the fibroblast-populated collagen lattice contraction (CLC) assay represents a validated approach to explore fibrotic processes *in vitro.* To evaluate the impact of Notch inhibition on RDEB-FB contractile ability, the CLC assay was performed on patients' fibroblasts treated with two commercially available GSIs, DAPT and PF-03084014 (nirogacestat) (see Table 1), in the presence or in the absence of TGF-β1 as contraction booster. In basal culture conditions, i.e. cells growth in the absence of TGF-β1 stimulation, the effects of PF-03084014 on cell contractility were modest but consistent in all RDEB-FB strains analyzed (Figure 4). In particular, PF-03084014 exhibited a striking inhibition of contractile properties in RDEB-FB1, which shares remarkable contractile abilities with RDEB-FB2, but seems to be more responsive to PF-03084014 stimulation as compared to its cognate cell strain (Figure 4). On the other hand, RDEB-FBs treated with DAPT didn't exhibit significant variations of the ability to contract the surrounding type I collagen matrix in the absence of TGF-β1 stimulation (data not shown). Conversely, in the presence of TGF-β1, RDEB-FBs treated with PF-03084014 showed a marked reduction of contractility (Fig. 5) whilst DAPT-dependent effects were also significant, although less conspicuous (Fig. 5B and Figure 6). The different aptitude of the two GSIs to lessen RDEB-FB contractility, and, in turn, to inhibit fibrotic processes *in vitro,* was unambiguously demonstrated when the same RDEB-FB strains (*i.e.* RDEB-FB3 and RDEB-FB4) were simultaneously subjected to the treatment with PF-03084014 or DAPT for 48 h, in the presence of TGF-β1 as contraction inducer (Fig. 5B). Finally, to corroborate the Notch involvement in RDEB-FB contractility, CLC assay was repeated on RDEB-FBs transfected with a short interfering RNA (siRNA) targeting JAG1 (si-JAG1). The results showed that the cell ability to contract the surrounding type I collagen matrix was reduced in JAG1-silenced RDEB-FBs with respect to cells transfected with a non-targeting scrambled control siRNA (Scramble), with and without TGF-β1 stimulation (Fig. 5D). Taken together, these findings support the role of the Notch signaling cascade as positive regulator of the contractile ability of activated fibroblasts, which is a distinctive trait of fibrosis. In addition, the superior effect of PF-03084014 in counteracting RDEB-FB contractility as compared to DAPT was proved.

### PF-03084014 counteracts migratory potential and proliferation rate of primary RDEB fibroblasts

As part of the tissue repair process, activated fibroblasts initially migrate to injury sites and start to proliferate and produce ECM components, mainly type I and III collagens. Once tissue integrity is re-established, myofibroblasts can return to their "low-activity state" via de-differentiation into fibroblasts and other precursor cells or, alternatively, undergo senescence or programmed cell death. However, in *in vitro* and *in vivo* disease models of fibrosis, myofibroblasts persist at the lesion site due to impaired self-clearance mechanisms or their excessive activation/proliferation [27,28]. In this study the effects of PF-03084014 and DAPT on the migratory ability and proliferation rate of RDEB-FBs were evaluated. As for cell migration, Notch pathway inhibition by both GSIs led to a significant decrease of RDEB-FB ability to migrate into the cell-free area with respect to non-treated cells (Fig. 7A). In parallel, the effects of GSIs on RDEB-FB proliferation ability were assessed by MTT assay on three different RDEB-FB strains treated with PF-03084014 or DAPT for 24 and 48 h. RDEB-FBs treated with DAPT didn't display variations in cell proliferation (Fig. 7B). Conversely, the treatment with PF-03084014 for 48 h determined a statistically significant reduction of RDEB-FB proliferation rate as compared to untreated cells (Fig. 7B), highlighting the greater effects of PF-03084014 with respect to DAPT, also in the context of cellular growth.

### RDEB-FBs treated with PF-03084014 show a reduction in TGF-β1 secretion and collagen deposition

To assess whether the inhibition of the Notch pathway can attenuate two hallmarks of tissue fibrosis, *i.e.* collagen deposition and TGF-β1 release by activated fibroblasts, the amount of ECM collagens and the concentration of TGF-β1 in acid-activated supernatants were quantified in RDEB-FBs treated with PF-03084014 and DAPT. As for TGF-β1 secretion, the amount of cytokine released by three different strains of RDEB-FBs in culture media was reduced by up to 60% in the presence of the GSI PF-03084014 (20 µM) with respect to control cells treated with DMSO and DAPT (20 µM) (DMSO at 48 h = 233.7 ± 79.9 pg/mL, 20 µM DAPT at 48 h = 250.4 ± 97.3 pg/mL, 20 µM PF-03084014 at 48 h = 99.6 ± 40.8 pg/mL) (Fig. 8A). In addition, RDEB-FBs treated with PF-03084014 showed a 50% reduction of pepsin-soluble collagens deposited into the ECM as compared to untreated cells, both in the absence or in the presence of TGF-β1 as inducer of collagen deposition (Fig. 8B). The results revealed also a DAPT-mediated reduction of collagen deposition in each experimental condition, though the difference was moderate if compared to the treatment with PF-03084014 (Fig. 8B). Finally, the impact of GSIs on the capability of RDEB-FBs to remodel a layer of type I collagen fibrils was investigated by an *in vitro* degradation assay [46,48]. In the presence of TGF-β1, RDEB-FBs treated with PF-03084014 exhibited an increased number and size of collagen degradation areas as compared to untreated controls (Fig. 8C). Although less marked, similar results were obtained in RDEB-FBs treated with DAPT (Fig. 8C).

### Pharmacological and short interfering RNA-mediated inhibition of Notch signaling pathway down-regulates myofibroblast markers

To evaluate the effects of Notch inhibition on fibroblast activation, the expression levels of a selection of prototypical contractility and pro-fibrotic markers were investigated by immunoblotting (IB) analysis and real-time PCR in RDEB-FBs treated with GSIs or transfected with a short interfering RNA targeting JAG1 (si-JAG1), in basal culture conditions and in the presence of TGF-β1. Despite inter-individual variations in gene expression levels, which are expected when dealing with primary cells from patients with different phenotypic manifestations and severity, IB analysis revealed that PF-03084014-treated RDEB-FBs showed the down-regulation of the prototypical contractile and fibrotic markers, including JAG1 (Fig. 9A), both in the absence and in the presence of TGF-β1 (Fig. 9A). Similar patterns of modulation were observed in RDEB-FBs treated with DAPT, although less marked (Figure 10). Accordingly, JAG1-silenced fibroblasts exhibited a significant reduction of α-SMA, CNN1, TAGLN, JAG1 and unprocessed NOTCH1 receptor (*i.e.* NOTCH full length, NOTCH FL) protein levels with respect to transfection controls, *i.e.* cells transfected with a scramble molecule (Scramble) (Fig. 9B). The effects of JAG1 silencing on the abundance of pro-fibrotic markers were more evident in RDEB-FBs stimulated with TGF-β1 (Fig. 9B), likely due to the TGF-β1-mediated induction of pro-fibrotic pathways, including Notch. Although with inter-individual variability, also the real-time PCR analysis confirmed the down-regulation of a selection of contractile/fibrotic markers, including α-SMA, CNN1, TAGLN and JAG1, in RDEB-FBs treated with PF-03084014 both in basal culture conditions (Fig. 9C) as well as in the presence of TGF-β1 (Fig. 9C). In addition, it was observed a context-dependent downregulation of mRNA levels of HES1 (hes family bHLH transcription factor 1), a transcriptional target of Notch, and of periostin (POST) and Pri-miR-143/145, which represent two validated pro-fibrotic genes [24,40] (Fig. 9C).

### References

1.Bardhan, A.; Bruckner-Tuderman, L.; Chapple, I.L.C.; Fine, J.D.; Harper, N.; Has, C.; Magin, T.M.; Marinkovich, M.P.; Marshall, J.F.; McGrath, J.A.; et al. Epidermolysis bullosa. Nat Rev Dis Primers 2020, 6, 78, doi:10.1038/s41572-020-0210-0.
2. Kridin, K.; Kneiber, D.; Kowalski, E. H.; Valdebran M.; Amber, K. T. Epidermolysis bullosa acquisita: A comprehensive review. Autoimmunity reviews, 2019, 18, 786-795, doi: 10.1016/j.autrev.2019.06.007
3.Has, C.; Bauer, J.W.; Bodemer, C.; Bolling, M.C.; Bruckner-Tuderman, L.; Diem, A.; Fine, J.D.; Heagerty, A.; Hovnanian, A.; Marinkovich, M.P.; et al. Consensus reclassification of inherited epidermolysis bullosa and other disorders with skin fragility. Br J Dermatol 2020, 183, 614-627, doi: 1 0.1111/bjd.18921.
4.Condorelli, A.G.; Dellambra, E.; Logli, E.; Zambruno, G.; Castiglia, D. Epidermolysis Bullosa-Associated Squamous Cell Carcinoma: From Pathogenesis to Therapeutic Perspectives. Int J Mol Sci 2019, 20, doi:10.3390/ijms20225707.
5.El Hachem, M.; Zambruno, G.; Bourdon-Lanoy, E.; Ciasulli, A.; Buisson, C.; Hadj-Rabia, S.; Diociaiuti, A.; Gouveia, C.F.; Hernandez-Martin, A.; de Lucas Laguna, R.; et al. Multicentre consensus recommendations for skin care in inherited epidermolysis bullosa. Orphanet J Rare Dis 2014, 9, 76, doi:10.1186/1750-1172-9-76.
6.Has, C.; El Hachem, M.; Buckova, H.; Fischer, P.; Friedova, M.; Greco, C.; Nevorankova, P.; Salavastru, C.; Mellerio, J.E.; Zambruno, G.; et al. Practical management of epidermolysis bullosa: consensus clinical position statement from the European Reference Network for Rare Skin Diseases. J Eur Acad Dermatol Venereol 2021, 35, 2349-2360, doi:10.1111/jdv.17629.
7. Kern, J. S.; Sprecher, E.; Fernandez, M. F.; Schauer, F.; Bodemer, C.; Cunningham, T.; Löwe, S.; Davis, C.; Sumeray, M.; Bruckner, A. L.; Murrell, D. F.; & EASE investigators. Efficacy and safety of Oleogel-S10 (birch triterpenes) for epidermolysis bullosa: results from the phase III randomized double-blind phase of the EASE study. Br J Dermatol 2023, 188, 12-21, doi:10.1093/bjd/ljac001.
8.Bruckner-Tuderman, L. Skin Fragility: Perspectives on Evidence-based Therapies. Acta Derm Venereol 2020, 100, adv00053, doi:10.2340/00015555-3398.
9.Kiritsi, D.; Dieter, K.; Niebergall-Roth, E.; Fluhr, S.; Daniele, C.; Esterlechner, J.; Sadeghi, S.; Ballikaya, S.; Erdinger, L.; Schauer, F.; et al. Clinical trial of ABCB5+ mesenchymal stem cells for recessive dystrophic epidermolysis bullosa. JCl Insight 2021, 6, doi:10.1172/jci.insight.151922.
10.Hou, P.C.; Wang, H.T.; Abhee, S.; Tu, W.T.; McGrath, J.A.; Hsu, C.K. Investigational Treatments for Epidermolysis Bullosa. Am J Clin Dermatol 2021, 22, 801-817, doi:10.1007/s40257-021-00626-3.
11.Gurevich, I.; Agarwal, P.; Zhang, P.; Dolorito, J.A.; Oliver, S.; Liu, H.; Reitze, N.; Sarma, N.; Bagci, I.S.; Sridhar, K.; et al. In vivo topical gene therapy for recessive dystrophic epidermolysis bullosa: a phase 1 and 2 trial. Nat Med 2022, 28, 780-788, doi:10.1038/s41591-022-01737-y.
12.Subramaniam, K.S.; Antoniou, M.N.; McGrath, J.A.; Lwin, S.M. The potential of gene therapy for recessive dystrophic epidermolysis bullosa. Br J Dermatol 2022, 186, 609-619, doi:10.1111/bjd.20910.
13.Hirsch, T.; Rothoeft, T.; Teig, N.; Bauer, J.W.; Pellegrini, G.; De Rosa, L.; Scaglione, D.; Reichelt, J.; Klausegger, A.; Kneisz, D.; et al. Regeneration of the entire human epidermis using transgenic stem cells. Nature 2017, 551, 327-332, doi:10.1038/nature24487.
14.Kueckelhaus, M.; Rothoeft, T.; De Rosa, L.; Yeni, B.; Ohmann, T.; Maier, C.; Eitner, L.; Metze, D.; Losi, L.; Secone Seconetti, A.; et al. Transgenic Epidermal Cultures for Junctional Epidermolysis Bullosa - 5-Year Outcomes. N Engl J Med 2021, 385, 2264-2270, doi:10.1056/NEJMoa2108544.
15.Eichstadt, S.; Barriga, M.; Ponakala, A.; Teng, C.; Nguyen, N.T.; Siprashvili, Z.; Nazaroff, J.; Gorell, E.S.; Chiou, A.S.; Taylor, L.; et al. Phase 1/2a clinical trial of gene-corrected autologous cell therapy for recessive dystrophic epidermolysis bullosa. JCI Insight 2019, 4, doi:10.1172/jci.insight. 130554.
16. Guide, S. V.; Gonzalez, M. E.; Ba 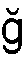 ci, I. S.; Agostini, B.; Chen, H.; Feeney, G.; Steimer, M.; Kapadia, B.; Sridhar, K.; Quesada Sanchez, L.; Gonzalez, F., Van Ligten, M., Parry, T. J., Chitra, S., Kammerman, L. A.; Krishnan, S.; & Marinkovich, M. P. Trial of Beremagene Geperpavec (B-VEC) for Dystrophic Epidermolysis Bullosa. New Engl J Med **2023,** *387*, 2211-2219, doi:10.1056/NEJMoa2206663.
17.Lodyga, M.; Hinz, B. TGF-beta1 - A truly transforming growth factor in fibrosis and immunity. Semin Cell Dev Biol 2020, 101, 123-139, doi:10.1016/j.semcdb.2019.12.010.
18.Akasaka, E.; Kleiser, S.; Sengle, G.; Bruckner-Tuderman, L.; Nystrom, A. Diversity of Mechanisms Underlying Latent TGF-beta Activation in Recessive Dystrophic Epidermolysis Bullosa. J Invest Dermatol 2021, 141, 1450-1460 e1459, doi:10.1016/j.jid.2020.10.024.
19.Titeux, M.; Pendaries, V.; Tonasso, L.; Decha, A.; Bodemer, C.; Hovnanian, A. A frequent functional SNP in the MMP1 promoter is associated with higher disease severity in recessive dystrophic epidermolysis bullosa. Hum Mutat 2008, 29, 267-276, doi:10.1002/humu.20647.
20.Odorisio, T.; Di Salvio, M.; Orecchia, A.; Di Zenzo, G.; Piccinni, E.; Cianfarani, F.; Travaglione, A.; Uva, P.; Bellei, B.; Conti, A.; et al. Monozygotic twins discordant for recessive dystrophic epidermolysis bullosa phenotype highlight the role of TGF-beta signalling in modifying disease severity. Hum Mol Genet 2014, 23, 3907-3922, doi:10.1093/hmg/ddu102.
21.Cianfarani, F.; De Domenico, E.; Nystrom, A.; Mastroeni, S.; Abeni, D.; Baldini, E.; Ulisse, S.; Uva, P.; Bruckner-Tuderman, L.; Zambruno, G.; et al. Decorin counteracts disease progression in mice with recessive dystrophic epidermolysis bullosa. Matrix Biol 2019, 81, 3-16, doi:10.1016/j.matbio.2018.12.001.
22.Atanasova, V.S.; Russell, R.J.; Webster, T.G.; Cao, Q.; Agarwal, P.; Lim, Y.Z.; Krishnan, S.; Fuentes, I.; Guttmann-Gruber, C.; McGrath, J.A.; et al. Thrombospondin-1 Is a Major Activator of TGF-beta Signaling in Recessive Dystrophic Epidermolysis Bullosa Fibroblasts. J Invest Dermatol 2019, 139, 1497-1505 e1495, doi:10.1016/j.jid.2019.01.011.
23.Vanden Oever, M.; Muldoon, D.; Mathews, W.; McElmurry, R.; Tolar, J. miR-29 Regulates Type VII Collagen in Recessive Dystrophic Epidermolysis Bullosa. J Invest Dermatol 2016, 136, 2013-2021, doi:10.1016/j.jid.2016.05.115.
24.Condorelli, A.G.; Logli, E.; Cianfarani, F.; Teson, M.; Diociaiuti, A.; El Hachem, M.; Zambruno, G.; Castiglia, D.; Odorisio, T. MicroRNA-145-5p regulates fibrotic features of recessive dystrophic epidermolysis bullosa skin fibroblasts. Br J Dermatol 2019, 181, 1017-1027, doi:10.1111/bjd.17840.
25.Hinz, B.; McCulloch, C.A.; Coelho, N.M. Mechanical regulation of myofibroblast phenoconversion and collagen contraction. Exp Cell Res 2019, 379, 119-128, doi:10.1016/j.yexcr.2019.03.027.
26.Pakshir, P.; Noskovicova, N.; Lodyga, M.; Son, D.O.; Schuster, R.; Goodwin, A.; Karvonen, H.; Hinz, B. The myofibroblast at a glance. J Cell Sci 2020, 133, doi:10.1242/jcs.227900.
27.Kato, K.; Logsdon, N.J.; Shin, Y.J.; Palumbo, S.; Knox, A.; Irish, J.D.; Rounseville, S.P.; Rummel, S.R.; Mohamed, M.; Ahmad, K.; et al. Impaired Myofibroblast Dedifferentiation Contributes to Nonresolving Fibrosis in Aging. Am J Respir Cell Mol Biol 2020, 62, 633-644, doi:10.1165/rcmb.2019-0092OC.
28.Fortier, S.M.; Penke, L.R.; King, D.; Pham, T.X.; Ligresti, G.; Peters-Golden, M. Myofibroblast dedifferentiation proceeds via distinct transcriptomic and phenotypic transitions. JCI Insight 2021, 6, doi:10.1172/jci.insight.144799.
29.Siebel, C.; Lendahl, U. Notch Signaling in Development, Tissue Homeostasis, and Disease. Physiol Rev 2017, 97, 1235-1294, doi:10.1152/physrev.00005.2017.
30.Condorelli, A.G.; El Hachem, M.; Zambruno, G.; Nystrom, A.; Candi, E.; Castiglia, D. Notch-ing up knowledge on molecular mechanisms of skin fibrosis: focus on the multifaceted Notch signalling pathway. J Biomed Sci 2021, 28, 36, doi:10.1186/s12929-021-00732-8.
31.Dees, C.; Tomcik, M.; Zerr, P.; Akhmetshina, A.; Horn, A.; Palumbo, K.; Beyer, C.; Zwerina, J.; Distler, O.; Schett, G.; et al. Notch signalling regulates fibroblast activation and collagen release in systemic sclerosis. Ann Rheum Dis 2011, 70, 1304-1310, doi:10.1136/ard.2010.134742.
32.Wen, J.; Liu, D.; Zhao, L. Small molecules targeting gamma-secretase and their potential biological applications. Eur J Med Chem 2022, 232, 114169, doi:10.1016/j.ejmech.2022.114169.
33.Moore, G.; Annett, S.; McClements, L.; Robson, T. Top Notch Targeting Strategies in Cancer: A Detailed Overview of Recent Insights and Current Perspectives. Cells 2020, 9, doi:10.3390/cells9061503.
34.Lanz, T.A.; Wood, K.M.; Richter, K.E.; Nolan, C.E.; Becker, S.L.; Pozdnyakov, N.; Martin, B.A.; Du, P.; Oborski, C.E.; Wood, D.E.; et al. Pharmacodynamics and pharmacokinetics of the gamma-secretase inhibitor PF-3084014. J Pharmacol Exp Ther 2010, 334, 269-277, doi:10.1124/jpet.110.167379.
35.Brodney, M.A.; Auperin, D.D.; Becker, S.L.; Bronk, B.S.; Brown, T.M.; Coffman, K.J.; Finley, J.E.; Hicks, C.D.; Karmilowicz, M.J.; Lanz, T.A.; et al. Design, synthesis, and in vivo characterization of a novel series of tetralin amino imidazoles as gamma-secretase inhibitors: discovery of PF-3084014. Bioorg Med Chem Lett 2011, 21, 2637-2640, doi:10.1016/j.bmcl.2010.12.118.
36.Skubitz, K.M. Biology and Treatment of Aggressive Fibromatosis or Desmoid Tumor. Mayo Clin Proc 2017, 92, 947-964, doi:10.1016/j.mayocp.2017.02.012.
37.Kummar, S.; O'Sullivan Coyne, G.; Do, K.T.; Turkbey, B.; Meltzer, P.S.; Polley, E.; Choyke, P.L.; Meehan, R.; Vilimas, R.; Horneffer, Y.; et al. Clinical Activity of the gamma-Secretase Inhibitor PF-03084014 in Adults With Desmoid Tumors (Aggressive Fibromatosis). J Clin Oncol 2017, 35, 1561-1569, doi: 1 0.1200/JCO.2016. 71.1994.
38.Takahashi, T.; Prensner, J.R.; Robson, C.D.; Janeway, K.A.; Weigel, B.J. Safety and efficacy of gamma-secretase inhibitor nirogacestat (PF-03084014) in desmoid tumor: Report of four pediatric/young adult cases. Pediatr Blood Cancer 2020, 67, e28636, doi:10.1002/pbc.28636.
39. Gounder, M.; Ratan, R.; Alcindor, T.; Schöffski, P.; van der Graaf, W. T.; Wilky, B. A.; Riedel, R. F.; Lim, A.; Smith, L. M.; Moody, S.; Attia, S.; Chawla, S.; D'Amato, G.; Federman, N.; Merriam, P.; Van Tine, B. A.; Vincenzi, B.; Benson, C.; Bui, N. Q.; Chugh, R.; ... Kasper, B. Nirogacestat, a γ-Secretase Inhibitor for Desmoid Tumors. New Engl J Med, 2023, 388, 898-912, doi:10.1056/NEJMoa2210140.
40.Chacon-Solano, E.; Leon, C.; Diaz, F.; Garcia-Garcia, F.; Garcia, M.; Escamez, M.J.; Guerrero-Aspizua, S.; Conti, C.J.; Mencia, A.; Martinez-Santamaria, L.; et al. Fibroblast activation and abnormal extracellular matrix remodelling as common hallmarks in three cancer-prone genodermatoses. Br J Dermatol 2019, 181, 512-522, doi:10.1111 /bjd.17698.
41.Rasul, S.; Balasubramanian, R.; Filipovic, A.; Slade, M.J.; Yague, E.; Coombes, R.C. Inhibition of gamma-secretase induces G2/M arrest and triggers apoptosis in breast cancer cells. Br J Cancer 2009, 100, 1879-1888, doi:10.1038/sj.bjc.6605034.
42.Lyons, R.M.; Keski-Oja, J.; Moses, H.L. Proteolytic activation of latent transforming growth factor-beta from fibroblast-conditioned medium. J Cell Biol 1988, 106, 1659-1665, doi:10.1083/jcb.106.5.1659.
43.Annes, J.P.; Munger, J.S.; Rifkin, D.B. Making sense of latent TGFbeta activation. J Cell Sci 2003, 116, 217-224, doi:10.1242/jcs.00229.
44.Murad, S.; Tajima, S.; Johnson, G.R.; Sivarajah, S.; Pinnell, S.R. Collagen synthesis in cultured human skin fibroblasts: effect of ascorbic acid and its analogs. J Invest Dermatol 1983, 81, 158-162, doi:10.1111/1523-1747.ep12543573.
45.Lareu, R.R.; Subramhanya, K.H.; Peng, Y.; Benny, P.; Chen, C.; Wang, Z.; Rajagopalan, R.; Raghunath, M. Collagen matrix deposition is dramatically enhanced in vitro when crowded with charged macromolecules: the biological relevance of the excluded volume effect. FEBS Lett 2007, 581, 2709-2714, doi:10.1016/j.febslet.2007.05.020.
46.Havemose-Poulsen, A.; Holmstrup, P.; Stoltze, K.; Birkedal-Hansen, H. Dissolution of type I collagen fibrils by gingival fibroblasts isolated from patients of various periodontitis categories. J Periodontal Res 1998, 33, 280-291, doi: 1 0.1111/j.1600-0765.1998.tb02201.x.
47.Livak, K.J.; Schmittgen, T.D. Analysis of relative gene expression data using real-time quantitative PCR and the 2(-Delta Delta C(T)) Method. Methods 2001, 25, 402-408, doi:10.1006/meth.2001.1262.
48.Zigrino, P.; Brinckmann, J.; Niehoff, A.; Lu, Y.; Giebeler, N.; Eckes, B.; Kadler, K.E.; Mauch, C. Fibroblast-Derived MMP-14 Regulates Collagen Homeostasis in Adult Skin. J Invest Dermatol 2016, 136, 1575-1583, doi:10.1016/j.jid.2016.03.036.

## Claims

1. Gamma-secretase inhibitor for use in the treatment of inherited epidermolysis bullosa and/or a fibrosis associated to inherited epidermolysis bullosa, wherein said gamma-secretase inhibitor inhibits the multiprotein complex of gamma-secretase through a direct mechanism.

2. Gamma-secretase inhibitor for use according to claim 1, wherein the inherited epidermolysis bullosa is chosen from the group consisting of recessive dystrophic epidermolysis bullosa, dominant dystrophic epidermolysis bullosa and junctional epidermolysis bullosa, preferably recessive dystrophic epidermolysis bullosa.

3. Gamma-secretase inhibitor for use according to any one of claims 1-2, wherein the fibrosis is recessive dystrophic epidermolysis bullosa-associated fibrosis.

4. Gamma-secretase inhibitor for use according to any one of claims 1-3, wherein said Gamma-secretase inhibitor is chosen from the group consisting of Nirogacestat, DAPT, RO492909, Semagacestat, BMS-906024, Avagacestat, MK-0752, preferably Nirogacestat.

5. Pharmaceutical composition comprising a gamma-secretase inhibitor, together with one or more excipients and/or adjuvants, for use in the treatment of inherited epidermolysis bullosa and/or a fibrosis associated to inherited epidermolysis bullosa, wherein said gamma-secretase inhibitor inhibits the multiprotein complex of gamma-secretase through a direct mechanism.

6. Pharmaceutical composition according to claim 5, for use according to claim 5, wherein the inherited epidermolysis bullosa is chosen from the group consisting of recessive dystrophic epidermolysis bullosa, dominant dystrophic epidermolysis bullosa and junctional epidermolysis bullosa, preferably recessive dystrophic epidermolysis bullosa.

7. Pharmaceutical composition according to any one of claims 5-6, for use according to any one of claims 5-6, wherein the fibrosis is recessive dystrophic epidermolysis bullosa-associated fibrosis.

8. Pharmaceutical composition according to any one of claims 5-7, for use according to any one of claims 5-7, wherein said Gamma-secretase inhibitor is chosen from the group consisting of Nirogacestat, DAPT, RO492909, Semagacestat, BMS-906024, Avagacestat, MK-0752, preferably Nirogacestat.

9. Pharmaceutical composition according to any one of claims 5-8, for use according to any one of claims 5-8, wherein said pharmaceutical composition further comprises a product chosen from the group consisting of anti-inflammatory drugs, losartan, Angiotensin (Ang)-(1-7) heptapeptide and Ang-(1-7)-based pharmaceutical products, such as TXA127 and oral formulations of the heptapeptide angiotensin-(1-7).

10. Combination of a gamma-secretase inhibitor with a product chosen from the group consisting of anti-inflammatory drugs, losartan, Angiotensin (Ang)-(1-7) heptapeptide, Ang-(1-7)-based pharmaceutical products, such as TXA127 and oral formulation of the heptapeptide angiotensin-(1-7), topical hydrogel formulations containing thymosin beta-4 (Tβ4), such as RGN-137, gels containing dry extract from birch bark, and topical gels containing Decorin, for separate or sequential use in the treatment of inherited epidermolysis bullosa and/or a fibrosis associated to inherited epidermolysis bullosa, wherein said gamma-secretase inhibitor inhibits the multiprotein complex of gamma-secretase through a direct mechanism.

11. Combination according to claim 10, for use according to claim 10, wherein the inherited epidermolysis bullosa is chosen from the group consisting of recessive dystrophic epidermolysis bullosa, dominant dystrophic epidermolysis bullosa (DDEB) and junctional epidermolysis bullosa (JEB), preferably recessive dystrophic epidermolysis bullosa.

12. Combination according to any one of claims 10-11, for use according to any one of claims 10-11, wherein the fibrosis is recessive dystrophic epidermolysis bullosa-associated fibrosis.

13. Combination according to any one of claims 10-12, for use according to any one of claims 10-12, wherein said Gamma-secretase inhibitor is chosen from the group consisting of Nirogacestat, DAPT, RO492909, Semagacestat, BMS-906024, Avagacestat, MK-0752, preferably Nirogacestat.

## Patentansprüche

1. Gamma-Sekretase-Inhibitor zur Verwendung bei der Behandlung von vererbter Epidermolysis bullosa und/oder einer mit vererbter Epidermolysis bullosa assoziierten Fibrose, wobei der Gamma-Sekretase-Inhibitor den Multiproteinkomplex der Gamma-Sekretase durch einen direkten Mechanismus hemmt.

2. Gamma-Sekretase-Inhibitor zur Verwendung gemäß Anspruch 1, wobei die vererbte Epidermolysis bullosa aus der Gruppe ausgewählt ist, die aus rezessiver dystropher Epidermolysis bullosa, dominanter dystropher Epidermolysis bullosa und junktionaler Epidermolysis bullosa besteht, vorzugsweise rezessiver dystropher Epidermolysis bullosa.

3. Gamma-Sekretase-Inhibitor zur Verwendung nach einem der Ansprüche 1 bis 2, wobei die Fibrose eine rezessive dystrophische Epidermolysis bullosa-assoziierte Fibrose ist.

4. Gamma-Sekretase-Inhibitor zur Verwendung nach einem der Ansprüche 1 bis 3, wobei der Gamma-Sekretase-Inhibitor ausgewählt ist aus der Gruppe bestehend aus Nirogacestat, DAPT, RO492909, Semagacestat, BMS-906024, Avagacestat, MK-0752, vorzugsweise Nirogacestat.

5. Pharmazeutische Zusammensetzung, die einen Gamma-Sekretase-Inhibitor zusammen mit einem oder mehreren Hilfsstoffen und/oder Adjuvantien umfasst, zur Verwendung bei der Behandlung von vererbter Epidermolysis bullosa und/oder einer Fibrose, die mit vererbter Epidermolysis bullosa assoziiert ist, wobei der Gamma-Sekretase-Inhibitor den MultiproteinKomplex der Gamma-Sekretase durch einen direkten Mechanismus hemmt.

6. Pharmazeutische Zusammensetzung nach Anspruch 5 zur Verwendung nach Anspruch 5, wobei die vererbte Epidermolysis bullosa ausgewählt ist aus der Gruppe bestehend aus rezessiver dystropher Epidermolysis bullosa, dominanter dystropher Epidermolysis bullosa und junktionaler Epidermolysis bullosa, vorzugsweise rezessiver dystropher Epidermolysis bullosa.

7. Pharmazeutische Zusammensetzung nach einem der Ansprüche 5 bis 6 zur Verwendung nach einem der Ansprüche 5 bis 6, wobei die Fibrose eine rezessive dystrophische Epidermolysis bullosa-assoziierte Fibrose ist.

8. Pharmazeutische Zusammensetzung nach einem der Ansprüche 5 bis 7 zur Verwendung nach einem der Ansprüche 5 bis 7, wobei der Gamma-Sekretase-Inhibitor ausgewählt ist aus der Gruppe bestehend aus Nirogacestat, DAPT, RO492909, Semagacestat, BMS-906024, Avagacestat, MK-0752, vorzugsweise Nirogacestat.

9. Pharmazeutische Zusammensetzung nach einem der Ansprüche 5 bis 8 zur Verwendung nach einem der Ansprüche 5 bis 8, wobei die pharmazeutische Zusammensetzung ferner ein Produkt umfasst, das aus der Gruppe ausgewählt ist, die aus entzündungshemmenden Arzneimitteln, Losartan, Angiotensin (Ang)-(1-7)-Heptapeptid und Ang-(1-7)-basierten pharmazeutischen Produkten, wie TXA127 und oralen Formulierungen des Heptapeptids Angiotensin-(1-7) besteht.

10. Kombination eines Gamma-Sekretase-Inhibitors mit einem Produkt, ausgewählt aus der Gruppe bestehend aus entzündungshemmenden Arzneimitteln, Losartan, Angiotensin (Ang)-(1-7)-Heptapeptid, Ang-(1-7)-basierten pharmazeutischen Produkten, wie TXA127 und oraler Formulierung des Heptapeptids Angiotensin-(1-7), topischen Hydrogel-Formulierungen, die Thymosin beta-4 (Tß4) enthalten, wie RGN-137, Gele, die Trockenextrakt aus Birkenrinde enthalten, und topische Gele, die Decorin enthalten, zur getrennten oder aufeinanderfolgenden Verwendung bei der Behandlung von vererbter Epidermolysis bullosa und/oder einer Fibrose, die mit vererbter Epidermolysis bullosa assoziiert ist, wobei der Gamma-Sekretase-Inhibitor den Multiproteinkomplex der Gamma-Sekretase durch einen direkten Mechanismus hemmt.

11. Kombination nach Anspruch 10 zur Verwendung nach Anspruch 10, wobei die vererbte Epidermolysis bullosa ausgewählt ist aus der Gruppe bestehend aus rezessiver dystropher Epidermolysis bullosa, dominanter dystropher Epidermolysis bullosa (DDEB) und junktionaler Epidermolysis bullosa (JEB), vorzugsweise rezessiver dystropher Epidermolysis bullosa.

12. Kombination nach einem der Ansprüche 10 bis 11 zur Verwendung nach einem der Ansprüche 10 bis 11, wobei die Fibrose eine rezessive dystrophe Epidermolysis bullosa-assoziierte Fibrose ist.

13. Kombination nach einem der Ansprüche 10 bis 12 zur Verwendung nach einem der Ansprüche 10 bis 12, wobei der Gamma-Sekretase-Inhibitor ausgewählt ist aus der Gruppe bestehend aus Nirogacestat, DAPT, RO492909, Semagacestat, BMS-906024, Avagacestat, MK-0752, vorzugsweise Nirogacestat.

## Revendications

1. Inhibiteur de la gamma-sécrétase pour le traitement de l'épidermolyse bulleuse héréditaire et/ou d'une fibrose associée à l'épidermolyse bulleuse héréditaire, dans lequel ledit inhibiteur de la gamma-sécrétase inhibe le complexe multiprotéique de la gamma-sécrétase par un mécanisme direct.

2. Inhibiteur de la gamma-sécrétase utilisé selon la revendication 1, dans lequel l'épidermolyse bulleuse héréditaire est choisie dans le groupe constitué par l'épidermolyse bulleuse dystrophique récessive, l'épidermolyse bulleuse dystrophique dominante et l'épidermolyse bulleuse jonctionnelle, de préférence l'épidermolyse bulleuse dystrophique récessive.

3. Inhibiteur de la gamma-sécrétase utilisé selon l'une des revendications 1 à 2, dans lequel la fibrose est une épidermolyse bulleuse dystrophique récessive associée à une fibrose.

4. Inhibiteur de la gamma-sécrétase utilisé selon l'une des revendications 1 à 3, dans lequel ledit inhibiteur de la gamma-sécrétase est choisi dans le groupe constitué par Nirogacestat, DAPT, RO492909, Semagacestat, BMS-906024, Avagacestat, MK-0752, de préférence Nirogacestat.

5. Composition pharmaceutique comprenant un inhibiteur de la gamma-sécrétase, ainsi qu'un ou plusieurs excipients et/ou adjuvants, pour le traitement de l'épidermolyse bulleuse héréditaire et/ou d'une fibrose associée à l'épidermolyse bulleuse héréditaire, dans laquelle ledit inhibiteur de la gamma-sécrétase inhibe le complexe multiprotéique de la gamma-sécrétase par le biais d'un mécanisme direct.

6. Composition pharmaceutique selon la revendication 5, pour l'utilisation selon la revendication 5, dans laquelle l'épidermolyse bulleuse héréditaire est choisie dans le groupe constitué par l'épidermolyse bulleuse dystrophique récessive, l'épidermolyse bulleuse dystrophique dominante et l'épidermolyse bulleuse jonctionnelle, de préférence l'épidermolyse bulleuse dystrophique récessive.

7. Composition pharmaceutique selon l'une des revendications 5 à 6, pour utilisation selon l'une des revendications 5 à 6, dans laquelle la fibrose est une épidermolyse bulleuse dystrophique récessive associée à une fibrose.

8. Composition pharmaceutique selon l'une des revendications 5 à 7, pour une utilisation selon l'une des revendications 5 à 7, dans laquelle l'inhibiteur de la Gamma-sécrétase est choisi dans le groupe constitué de Nirogacestat, DAPT, RO492909, Semagacestat, BMS-906024, Avagacestat, MK-0752, de préférence Nirogacestat.

9. Composition pharmaceutique selon l'une des revendications 5 à 8, pour l'utilisation selon l'une des revendications 5 à 8, dans laquelle ladite composition pharmaceutique comprend en outre un produit choisi dans le groupe constitué des médicaments anti-inflammatoires, du losartan, de l'heptapeptide angiotensine (Ang)-(1-7) et des produits pharmaceutiques à base d'Ang-(1-7), tels que le TXA127 et les formulations orales de l'heptapeptide angiotensine-(1-7).

10. Combinaison d'un inhibiteur de la gamma-sécrétase avec un produit choisi dans le groupe constitué par les médicaments anti-inflammatoires, le losartan, l'heptapeptide angiotensine (Ang)-(1-7), les produits pharmaceutiques à base d'Ang-(1-7), tels que le TXA127 et la formulation orale de l'heptapeptide angiotensine-(1-7), les formulations topiques d'hydrogel contenant de la thymosine bêta-4 (Tβ4), telles que le RGN-137, gels contenant un extrait sec d'écorce de bouleau et gels topiques contenant de la décorine, pour une utilisation séparée ou séquentielle dans le traitement de l'épidermolyse bulleuse héréditaire et/ou d'une fibrose associée à l'épidermolyse bulleuse héréditaire, dans laquelle ledit inhibiteur de la gamma-sécrétase inhibe le complexe multiprotéique de la gamma-sécrétase par le biais d'un mécanisme direct.

11. Combinaison selon la revendication 10, pour utilisation selon la revendication 10, dans laquelle l'épidermolyse bulleuse héréditaire est choisie dans le groupe constitué par l'épidermolyse bulleuse dystrophique récessive, l'épidermolyse bulleuse dystrophique dominante (DDEB) et l'épidermolyse bulleuse jonctionnelle (JEB), de préférence l'épidermolyse bulleuse dystrophique récessive.

12. Combinaison selon l'une des revendications 10 à 11, pour utilisation selon l'une des revendications 10 à 11, dans laquelle la fibrose est une épidermolyse bulleuse dystrophique récessive associée à une fibrose.

13. Combinaison selon l'une des revendications 10 à 12, pour utilisation selon l'une des revendications 10 à 12, dans laquelle ledit inhibiteur de la Gamma-sécrétase est choisi dans le groupe constitué de Nirogacestat, DAPT, RO492909, Semagacestat, BMS-906024, Avagacestat, MK-0752, de préférence Nirogacestat.
